# EUROPEAN PATENT SPECIFICATION

(11) **EP 3 194 417 B1**
(45) Date of publication and mention of the grant of the patent: **28.10.2020**
(21) Application number: 15843046.2
(22) Date of filing: 17.09.2015
(51) Int. Cl.: C07J 15/00, A61K 31/56, A61P 25/00

(54) **ENT-19-NORPROGESTERONE FOR USE IN THE TREATMENT OF TRAUMATIC BRAIN INJURY**
ENT-19-NORPROGESTERON ZUR BEHANDLUNG VON HIRNTRAUMA
ENT-19-NORPROGESTERONE POUR LE TRAITEMENT DES LÉSIONS CÉRÉBRALES TRAUMATIQUES

(30) Priority: 17.09.2014 US 201462051898 P; 18.09.2014 US 201462052457 P
(43) Date of publication of application: 26.07.2017
(73) Proprietor: Prevacus, Inc., Tallahassee, FL 32308 (US)
(72) Inventor: LEVY, Daniel E., San Mateo, CA 94402 (US)
(74) Representative: Loo, Chi Ching
(86) International application number: PCT/US2015/050633
(87) International publication number: WO 2016/044559

(56) References cited:
- WO-A1-96/40727
- WO-A1-2013/056181
- WO-A1-2016/209847
- WO-A2-2009/108804
- CN-A- 103 073 612
- FR-A1- 2 097 045
- US-A- 3 248 390
- JON NILSEN ET AL: "Impact of Progestins on Estrogen-Induced Neuroprotection: Synergy by Progesterone and 19-Norprogesterone and Antagonism by Medroxyprogesterone Acetate", ENDOCRINOLOGY, vol. 143, no. 1, 1 January 2002 (2002-01-01), pages 205-212, XP55522009, US ISSN: 0013-7227, DOI: 10.1210/endo.143.1.8582
- VANLANDINGHAM J W ET AL: "The enantiomer of progesterone acts as a molecular neuroprotectant after traumatic brain injury", NEUROPHARMACOLOGY, PERGAMON PRESS, OXFORD, GB, vol. 51, no. 6, 1 November 2006 (2006-11-01), pages 1078-1085, XP028079723, ISSN: 0028-3908, DOI: 10.1016/J.NEUROPHARM.2006.07.015 [retrieved on 2006-11-01]

## Description

### Cross-Reference To Related Application

This application claims priority to and the benefit of United States Application No. 62/051,898, filed on September 17, 2014 and of United States Application No. 62/052,457 filed September 18, 2014.

### Field of the Invention

The present invention relates to : the compound of formula J, namely ent-19-norprogesterone, compositions thereof as well as this compound for treating, minimizing and/or preventing traumatic brain injury (TBI), including severe TBI, moderate TBI, and mild TBI, including concussions.

### Background

Today it is believed that more than 1.5 million people experience a traumatic brain injury (TBI) each year in the United States. Of those affected with TBI, it is thought that at least about 75 percent sustain mild traumatic brain injury or MTBI, as opposed to moderate or severe TBI. Even though these injuries are defined as mild, MTBI may cause long-term or permanent impairments and disabilities. Many people with MTBI have difficulty returning to routine, daily activities and may be unable to return to work for many weeks or months. In addition to the human toll of these injuries, MTBI costs the U.S. approximately $17 billion each year. See Report to Congress on Mild Traumatic Brain Injury in the United States: Steps to Prevent a Serious Public Health Problem, September 2003, available at http://www.cdc.govincipc/pub-res/mtbi/mtbireport.pdf. See also http://www.cdc.gov/traumaticbraininjury/get_the_facts.html; Faul M, Xu L, Wald MM, Coronado VG. Traumatic brain injury in the United States: emergency department visits, hospitalizations, and deaths. Atlanta (GA): Centers for Disease Control and Prevention, National Center for Injury Prevention and Control; 2010; Thurman D, Alverson C, Dunn K, Guerrero J, Sniezek J. Traumatic brain injury in the United States: a public health perspective. J Head Trauma Rehabil, 14(6):602-615 (1999); Injury Prevention & Control: Traumatic Brain Injury, Traumatic Brain Injury in the United States: Fact Sheet, available at Centers for Disease Control and Prevention at http://www.cdc.gov/traumaticbraininjury/get_the_facts.html and http://www.cdc.gov/TraumaticBrainlnjury/index.html; National Hospital Discharge Survey (NHDS), 2010; National Hospital Ambulatory Medical Care Survey (NHAMCS), 2010; National Vital Statistics System (NVSS), 2010; and Finkelstein E, Corso P, Miller T and associates. The Incidence and Economic Burden of Injuries in the United States. New York (NY): Oxford University Press; 2006; and Coronado, McGuire, Faul, Sugerman, Pearson. The Epidemiology and Prevention of TBI (in press) 2012.

TBI amongst U.S. military personnel is also a critically important health concern especially for veterans in the Operation Iraqi Freedom (01F) and Operation Enduring Freedom (OEF). According to a Defense and Veterans Brain Injury Center (DVBIC) analysis of surveillance data released by the Department of Defense (DoD), 33,149 U.S. military personnel were diagnosed with a TBI in 2011 alone. This number included service members (SMs) in the Army, Navy, Marine Corps, Air Force, and from the active duty and reserve components of the National Guard. See U.S. Dept. of Defense: http://www.health.mil/Research/TBI_Numbers.aspx. The U.S. Department of Veterans Affairs (VA) estimates that of the 771,874 veterans who sought care from a VA Medical Center from the start of OEF in October 1, 2001 to December 31, 2011, a total of 59,218 veterans were evaluated or treated for a condition possibly related to a TBI. See U.S. Dept. of Veterans Affairs, 2012 available at http://www.publichealth.va.govidocsiepidemiology/healthcare-iutilization-report-fy2012-qtr1.pdf.

TBI is a nondegenerative, noncongenital insult to the brain that can result from a bump, blow or jolt to the head or a penetrating head injury that disrupts the normal function of the brain possibly leading to permanent or temporary impairment of cognitive, physical, and psychosocial functions, with an associated diminished or altered state of consciousness. Not all blows or jolts to the head can cause a TBI. The severity of a TBI can range from "mild" to "severe". A "mild TBI" is characterized as a brief change in mental status or consciousness, whereas a "severe TBI" is characterized as an extended period of unconsciousness or memory loss after the injury. See http://www.cdc.govitraumaticbraininjury/get_the facts.html. See also Centers for Disease Control and Prevention (CDC), National Center for Injury Prevention and Control. Report to Congress on mild traumatic brain injury in the United States: steps to prevent a serious public health problem. Atlanta (GA): Centers for Disease Control and Prevention, 2003.

The Glasgow Coma Scale (GCS) defines the severity of a TBI within 48 hours of injury. Thus, as used herein, moderate to severe brain injuries are defined as follows:
- Moderate brain injury is defined as a brain injury resulting in a loss of consciousness from 20 minutes to 6 hours and a Glasgow Coma Scale of 9 to 12. See http://www.traumaticbraininjury.com/symptoms-of-tbi/severe-tbi-symptoms/;
- Severe brain injury is defined as a brain injury resulting in a loss of consciousness of greater than 6 hours and a Glasgow Coma Scale of 3 to 8. See http://www.traumaticbraininjury.com/symptoms-of-tbi/severe-tbi-symptoms/;
- Mild traumatic brain injury (mTBI) is defined as the result of the forceful motion of the head or impact causing a brief change in mental status (confusion, disorientation or loss of memory) or loss of consciousness for less than 30 minutes. While MRI and CAT scans are often normal, a person with a mild TBI may remain conscious or may experience a loss of consciousness for a few seconds or minutes. Other symptoms of mild TBI include headache, confusion, difficulty thinking, lightheadedness, dizziness, blurred vision or tired eyes, ringing in the ears, bad taste in the mouth, fatigue or lethargy, frustration, a change in sleep patterns, behavioral or mood swings, memory problems, concentration, attention, or thinking. See http://www.traumaticbraininjury.com/symptoms-of-tbi/mild-tbi-symptoms/.

A person with a moderate or severe TBI may present these same symptoms, but may also present a headache that gets worse or does not go away, repeated vomiting or nausea, convulsions or seizures, an inability to awaken from sleep, dilation of one or both pupils of the eyes, slurred speech, weakness or numbness in the extremities, loss of coordination, and increased confusion, restlessness, or agitation. See http://www.ninds.nih.gov/disorders/tbi/tbi.htm

Today, most TBIs that occur each year are mild, commonly called concussions. See http://www.cdc.gov/traumaticbraininjury/get_the_facts.html. See also Centers for Disease Control and Prevention (CDC), National Center for Injury Prevention and Control. Report to Congress on mild traumatic brain injury in the United States: steps to prevent a serious public health problem. Atlanta (GA): Centers for Disease Control and Prevention; 2003. See also Injury Prevention & Control: Traumatic Brain Injury, Traumatic Brain Injury in the United States: Fact Sheet, available at Centers for Disease Control and Prevention, available at http://www.cdc.gov/traumaticbraininjury/get_the_facts.html. See also http://www.cdc.goviTraumaticBrainInjury/index.html. Thus, it is currently believed that concussion is the most common type of traumatic brain injury.

A concussion is a type of traumatic brain injury (TBI) caused by a bump, blow or jolt to the head with a temporary loss of brain function. Concussions can also occur from a fall or a blow to the body that causes the head and brain to rattle or move quickly back and forth. See http://www.cdc.goviconcussion/pdf/Fact_Sheet_ConcussTBI-a.pdf. See also Facts about Concussion and Brain Injury at http://www.cdc.goviconcussion/pdf/Fact_Sheet_ConcussTBI-a.pdf. Concussions are defined as a traumatically induced transient disturbance of brain function and involves a complex pathophysiological process and are a subset of MTBI, which are generally self-limited and at the less-severe end of the brain injury spectrum. See Harmon KG et al.: American Medical Society for Sports Medicine position statement: concussion in sport. Br J Sports Med. 47(3):184 (Feb., 2013).

It has been estimated that as many as 3.8 million concussions occur in the U.S.A. per year during competitive sports and recreational activities; however, as many as 50% of the concussions may go unreported. See Harmon KG et al.: American Medical Society for Sports Medicine position statement: concussion in sport. Br J Sports Med. 47(3):184 (Feb., 2013). In addition, concussion is big business in football in the U.S.A. In view of the fact that there are about 1,700 players in the NFL, about 66,000 student athletes playing college football, about another 1.1 million high school football players and approximately 250,000 youths who participate in Pop Warner football, there is a demand to find solutions to reducing risks associated with concussions, "...whose terrifying consequences regularly tear across the sports pages. And a wave of companies offering diagnostic tools and concussion treatments are just as eager to sell them a peace of mind." See Peter Keating: Concussion test may not be panacea - ImPACT sells tests and training to thousands, but some question program's validity, ESPN The Magazine, August 12, 2012 available at http://espn.go.com/espn/otl/story/ /id/8297794/neuropsychological-testing-concussions-not-panacea.

According to the Centers for Disease Control and Prevention, most people with a concussion recover quickly and fully. However, for some people, symptoms can last for days, weeks, or longer. In general, recovery may be slower among older adults, young children and teens. Those who have had a concussion in the past are also at risk of having another one and may find that it takes longer to recover if they have another concussion. Symptoms of concussion usually fall into four categories:

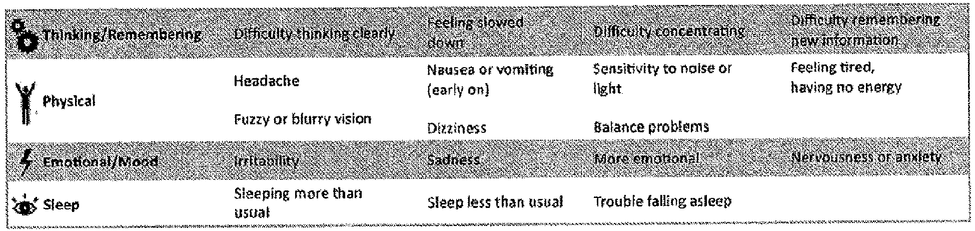

See http://www.cdc.gov/concussion/pdf/Fact_Sheet_ConcussTBl-a.pdf.

The terms mild brain injury, mild traumatic brain injury (MTBI), mild head injury (MHO, minor head trauma, and concussion may be used interchangeably. See National Center for Injury Prevention and Control. Report to congress on mild traumatic brain injury in the United States: Steps to prevent a serious public health problem. Atlanta, GA: Centers for Disease Control and Prevention (2003), Petchprapai N, Winkelman C: Mild traumatic brain injury: determinants and subsequent quality of life. A review of the literature. Journal of Neuroscience Nursing, 39 (5):260-72 (2007). See also Guidelines for Mild Traumatic Brain Injury and Persistent Symptoms available at http://onf.org/system/attachments/60/original/Guidelines_for_Mild Traumatic_Brain_Injury_and_Persistent_Symptoms. Although the term "concussion" is still used in sports literature as interchangeable with "MHI" or "MTBI", the general clinical medical literature now uses "MTBI" instead. See Barth JT, Varney NR, Ruchinskas RA, Francis JP: Mild head injury: The new frontier in sports medicine. In Varney NR, Roberts RJ. The Evaluation and Treatment of Mild Traumatic Brain Injury. Hillsdale, New Jersey: Lawrence Erlbaum Associates, pp. 85-6. (1999); and http://en.wikipedia.org/wiki/Concussion. Nonetheless, even though the terms are used interchangeably, a "concussion" is a subset of "MTBI". See Harmon KG et al.: American Medical Society for Sports Medicine position statement: concussion in sport. Br J Sports Med. 47(3):184 (Feb., 2013).

Progesterone is a C-21 steroid hormone. The chemical structure for progesterone is as follows:

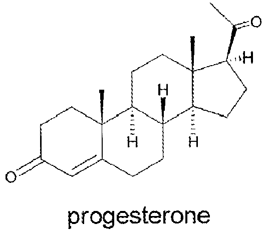

Progesterone is a progestogen, and it is one of the major naturally occurring human progestogens. Progesterone is involved in the female menstrual cycle, pregnancy and embryogenesis of humans and other species. Progesterone is naturally produced by the ovaries of mammals, but can also be produced by some plants and yeast.

19-Norprogesterone is a C-20 steroid hormone. The chemical structure for 19-Norprogesterone is as follows:

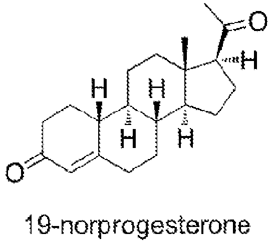

19-Norprogesterone is believed to be a potent progesten with mineralcorticoid properties and high affinity for the progesterone receptor. See Paris J, Botella J, Fournau P, Bonnet P, Thevenot R: Extinction of mineralocorticoid effects in 19-norprociesterone derivatives: structure-activity relationships; J. Pharmacol. Exp. Ther. 243 (1): 288-91 (1987); and Botella, J. et al: Structure-activity and structure-affinity relationships of 19-nor-progesterone derivatives in rat uterus. J Endocrinological Investigation. 13(11):905-910 (1990).

19-Norprogesterone is a member of the family of 19-nor-corticosteroids that is produced in extra-adrenal tissue in biologically relevant quantities. Levels of this class of steroids are known to be increased and possibly pathogenic in certain states of human hypertension. See Melby JC, Dale SL, Holbrook M, Griffing GT: 19-Nor-corticosteroids in experimental and human hypertension. Clin Exp Hypertens A; 4 (9¬10):1851-67 (1982).

The use of progesterone and its analogues have many medical applications, both to address acute situations and to address the long-term decline of natural progesterone levels. Other uses of progesterone include, for example, the prevention of preterm birth, to control anovulatury bleeding, to increase skin elasticity and bone strength, and to treat multiple sclerosis.

Today, there is a belief that progesterone may be useful for the treatment of traumatic brain injury (TBI), which may result in substantial and sustained improvements in cytologic, morphologic, and functional outcomes. See Schumacher M, Weill-Engerer S, Liere P, et al.: Steroid hormones and neurosteroids in normal and pathological aging of the nervous system. Prog Neurobiol; 71:3-29 (2003). For example, it has been reported that the administration of progesterone following brain injury may limit brain damage, reduce loss of neuronal tissue and improve functional recovery. See Goss CW, Hoffman SW, Stein DG. Behavioral effects and anatomic correlates after brain injury: a progesterone dose-response study. Pharmacol Biochem Behay. 76: 231-42 (2003). It has also been reported that progesterone may reduce poor outcomes following traumatic brain injury by inhibiting inflammatory factors (TNF-a and IL-13) and subsequently reducing brain edema. See Pan, D., et al.: Biomed Environ Sci. 20:432¬438 (2007); and Jiang, C., et al.: Inflamm Res. 58:619-624 (2009). Still further, it has been reported that progesterone-treated rats may demonstrate improvements on a Neurological Severity Score (test for motor and cognitive functioning) following traumatic brain injury. See Roof, R. L., et al.: Restor Neurol Neurosci. 4:425-427 (1992).

In addition, it has been reported that progesterone may effectively attenuate edema in both rodent sexes following injury (Djebaili, M., et al.: J Neurotrauma. 22, 106-118 (2005). Administering progesterone or its derivative allopregnanolone (ALLO) also results in a decrease of the presence of the factors of cell death (caspase-3) and gliosis (GFAP), Cutler, S. M., et al.: J Neurotrauma. 24:1475-1486 (2007), following injury, VanLandingham, J. W., et al.: Neurosci Lett. 425:94-98 (2007); Wright, D. W., et al.: Ann Emerg Med. 49:391-402, 402 e391-392 (2007). See also, Progesterone for the Treatment of Traumatic Brain Injury (ProTECT III), ClinicalTrials.gov Identifier:NCT00822900 and http://acutecareresearch.org/studies/current/progesterone-treatment-tbi-protect-iii; Efficacy and Safety Study of Intravenous Progesterone in Patients With Severe Traumatic Brain Injury (SyNAPSe), ClinicalTrials.gov Identifier:NCT01143064; Progesterone Treatment of Blunt Traumatic Brain Injury, ClinicalTrials.gov Identifier:NCT00048646; Blood Tests to Study Injury Severity and Outcome in Traumatic Brain Injury Patients (BioProTECT), ClinicalTrials.gov Identifier:NCT01730443. See further, ProTECT™III at http://www.protectiii.com/; and http://em.emorv.edu/protect/; and http://clinicaltrials.qov/show/NCT00822900. See also Progesterone for Traumatic Brain Injury Tested in Phase III Clinical Trial at http://www.sciencedaily.com/releases/2010/02/100219204407.htm. Still further, see BHR Pharma Investigational Traumatic Brain Injury Treatment Receives European Medicines Agency Orphan Medicinal Product Designation at http://synapse-trial.com/downloads/PREMAOrphan.pdf.

More recently, it has been reported that "...progesterone given to both male and female laboratory rats and mice can cross the blood-brain barrier...and reduce edema levels after TBI...; reduce lipid peroxidation and isoprostanes, which, in turn, contribute to postinjury ischemic conditions...; generate metabolites that reduce proapoptotic and increase antiapoptotic enzymes...and the expression of proinflammatory genes and their protein products...; influence the expression of aquaporins implicated in the resolution of edema...; in different models of cerebral ischemia, significantly reduce the area of necrotic cell death and improve behavioral outcomes...; protect neurons distal to the injury that would normally die...; enhance ligodendrocyte-induced remyelination in young and old rats with demyelinating disorders...; and produce significant sparing of cognitive, sensory, and spatial learning performance after bilateral medial frontal cortex injury...Progesterone has been shown to have beneficial effects in 22 different injury models; a number of extensive reviews discuss these data...To date, most research on progesterone and its metabolites has focused on the treatment of TBI...This line of research originated when researchers...found that, after bilateral contusion injury to the medial frontal cortex in young adult male and female rats, 5 days of treatment with progesterone significantly improved spatial learning and sensory performance, compared with controls given injections of the vehicle alone. The first successful clinical trial for the treatment of TBI in more than 30 years of research was recently completed. This National Institute of Neurological Disorders and Stroke (NINDS)-sponsored phase 2a single-center clinical trial for progesterone in the treatment of moderate-to-severe adult TBI...found that the mortality rate among patients given progesterone IV for 3 days after the injury was less than half that among control subjects given the standard-of-practice care but no hormone (13.6% vs 30.4%). Thirty-day functional outcomes for moderately injured patients in the progesterone group were significantly better than those for the placebo group [and]...that a National Institutes of Health-appointed data safety monitoring board found no serious adverse events attributable to progesterone treatment in this trial. A second independent randomized double-blind study from China examined 159 patients with severe TBI given a course of intramuscular injections of progesterone for 5 days. The investigators reported very similar beneficial outcomes on morbidity and mortality at both 30 days and 6 months after injury, again without any serious adverse events caused by the treatment..." See D.G. Stein and I. Sayeed: Is Progesterone Worth Consideration as a Treatment for Brain Injury? AJR (194):20-22 (January 2010).

In about June 2010, BHR Pharma initiated the SyNAPSe® study (Study of the Neuroprotective Activity of Progesterone in Severe Traumatic Brain Injuries) to study the effectiveness of an intravenous progesterone infusion formula. See http://www.synapse-trial.comi; http://www.besinscriticalcare.conn/progesterone-research/; and http://em.emory.edu/protect/. Nonetheless, it is reported that "BHR-100 must be administered within eight hours of the TBI and infused continuously over five days...The SyNAPSe® study's Independent Data and Safety Monitoring Board (DSMB) has released six analyses of the trial's safety data over the course of the study, concluding each time that SyNAPSe® should continue to its intended completion...The DSMB's formal interim analysis of primary six-month efficacy data from 400 SyNAPSe patients, conducted in January 2013, concluded that there was no reason to stop the study for futility... [and] The SyNAPSe® study is endorsed by the American Brain Injury Consortium (ABIC) and the European Brain Injury Consortium (EBIC)." See http://www.besinscriticalcare.conn/progesterone-research/. See also, BHR Pharma SyNAPSe® Trial DSMB Data Analyses Determine No Safety Issues; Study Should Continue to Conclusion at http://www.prnewswire.conninews-releases/bhr-pharnna-synapse-trial-dsnnb-data-analyses-determine-no-safety-issues-study-should-continue-to-conclusion-187277871.htnnl.

19-norprogesterone and its analogs may have medical applications. For example, this class of compounds is believed to facilitate axon remylination. See Hussain R, EI-Etr M, Gaci 0, Rakotomamonjy J, Macklin WB, Kumar N, Sitruk-Ware R, Schumacher M, Ghoumari AM: "Progesterone and Nestorone facilitate axon remyelination: a role for progesterone receptors", Endocrinology, 152 (10): 3820-31 (2011). Additionally, this class of compounds has been studied as potential oral contraceptives. See, e.g., Mueck AO, Sitruk-Ware R.: "Nomegestrol acetate, a novel progestogen for oral contraception", Steroids, 76 (6): 531-9 (2011). Additional useful activities may include inhibition of apoptosis. See Dressing GE, Pang Y, Donq J, Thomas P.: "Progestin signaling through mPRa in Atlantic croaker granulosa/theca cell cocultures and its involvement in progestin inhibition of apoptosis", Endocrinodogy_151, (12): 5916-26 (2010).

Progesterone exists in a non-naturally occurring enantiomeric form known as ent-progesterone:

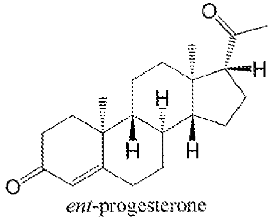

*ent-*Progesterone is believed to have equal efficacy to natural progesterone in reducing cell death, brain swelling, and inflammation while the enantiomer has three times the antioxidant activity of racemate under certain conditions. Similarly, *ent*-Progesterone is believed to have fewer sexual side effects such as suppression of spermatogenesis; inhibition of the conversion of testosterone to dihydrotestosterone; reduction in the size of the testes, epididymis, and leydig cells; and no hyper-coagulative risk as may be seen with natural progesterone. In addition, utilities for *ent*-progesterone have been described in U.S. Patent Application No. 13/645,881, which was filed on October 5, 2012 and is entitled "Nasal Delivery Mechanism for Prophylatic and Post-Acute Use for Progesterone and/or Its Enantiomer for Use in Treatment of Mild Traumatic Brain Injuries, U.S. Patent Application No. 13/645,854, which was filed on October 12, 2012 and is entitled "Prophylactic and Post-Acute Use of Progesterone and Its Enantiomer to Better Outcomes Associated with Concussion," and U.S. Patent Application No. 13/645,925, which was filed on October 12, 2012 and is entitled "Prophylactic and Post- 15 Acute Use of Progesterone in Conjunction with Its Enantiomer for Use in Treatment of Traumatic Brain Injuries. See also VanLandingham et al.: The enantiomer of progesterone acts as a molecular neuroprotectant after traumatic brain injury, Neuropharmacology, 51:1078-1085 (2006).

Due to side effects associated with long term progesterone treatments, it is believed that progesterone is not suitable for long-term chronic administration for indications outside of hormone replacement therapy and contraception. Even though there is a current belief that intravenous progesterone may be useful for the treatment of moderate to severe traumatic brain injury (TBI), MTBI in the U.S. population, including among those who served in the military, is a public health problem, the magnitude and impact of which are underestimated by current civilian and military surveillance systems. There is no doubt that much research is needed to determine the full magnitude of MTBI, including concussions, to identify preventable and modifiable risk factors, develop and test strategies to reduce MTB's in civilian and military life, and improve health and social outcomes and quality of life for those who sustain these injuries. Thus, there is a need for novel MTBI treatments that are effective, that can be conveniently administered on demand, that are tissue-specific and/or that do not induce side effects, such as those commonly associated with progesterone or the reproductive system.

### Summary of the Invention

In brief, it is believed that the present invention overcomes many of the disadvantages and shortcomings associated with the current state of mild traumatic brain injury (MTBI) treatment through the discovery of *ent-19-*norprogesterone, compositions and this compound for methods of use that are believed to be effective in the treatment of MTBI, including concussions a subset thereof, that can be administered either in accordance with a prescribed treatment regimen or conveniently on demand. Quite remarkably, the *ent-19-* norprogesterone of the present invention is believed to be tissue-specific and/or do not induce side effects, such as those associated with progesterone or the reproductive system. Uniquely, the *ent-19-* norprogesterone of the present invention can be conveniently administered by any route of administration, especially topically, e.g., pernasally, buccally and/or sublingually, on demand to deliver an effective amount to effectively and/or prophylactically treat and or prevent MTBI. The compound of the invention and compositions thereof as contemplated by the present invention are believed to be tissue-specific in the brain for treating MTBI and/or do not induce side effects commonly associated with progesterone or the reproductive system.

The C-20 steroid compound of the present invention is ent-19-norprogesterone. Ent-19-norprogesterone has a molecular formula of C₂₀ H₂₈ O₂ and a molar mass of 300.435 g/mol. The chemical names for ent-19-norprogesterone include ent-19- norpregn-4-ene-3,20-dione. The chemical structure of ent-norprogesterone is as follows:

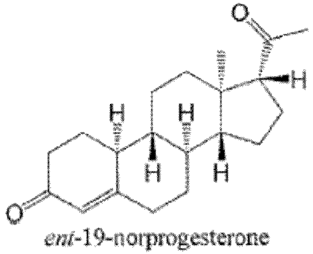

In accordance with the present invention, the *ent-19-* norprogesterone is believed to be useful for treating, minimizing and/or preventing neuronal damage, such as neuronal damage resulting from various injuries involving TBI, whether the TBI is mild, moderate or severe. An especially preferred treatment in accordance with the present invention is treatment of MTBI, including a concussion, with ent-19-norprogesterone.

The compound of the invention, *ent-19-* norprogesterone may be administered as a single therapeutic agent.

It is further contemplated that the *ent-19-* norprogesterone can be administered through routes of administration that include, e.g., oral, sublingual, intravenous, intraperitoneal, subcutaneous, intramuscular, ocular, otic, intranasal, topical, transdermal and rectal routes of administration. The present invention further envisions that the *ent-19-*norprogesterone can be formulated into a novel composition or admixture and administered in the form of, e.g., a tablet, capsule, gelcap, caplet, powder, granule, liquid, solution, suspension, dispersion, pellet, bead, eyedrop, gel, cream, ointment, salve, balm, lotion or suppository. Still further, it is envisioned that the *ent-19-* norprogesterone may be administered as a formulation that is swallowed, injected, infused, inhaled, applied transdermally or topically, such as applied to the skin, eye, ear, nose, mucosal membrane or any other membrane or inserted into the rectum. Nonetheless, it should be understood by those versed in the art that preferred routes of administration to treat TBI, especially MTBI, as contemplated by the present invention, is the pernasal, inhalation or injection routes of administration.

### Detailed Descrintion

By way of illustrating and providing a more complete appreciation of the present invention and many of the attendant advantages thereof, the following detailed description and examples are given concerning the : compound of the invention, compositions, and methods of manufacture and uses thereof of the present invention.

As used in the description of the invention and the appended claims, the singular forms "a", "an" and "the" are used interchangeably and intended to include the plural forms as well and fall within each meaning, unless the context clearly indicates otherwise. Also, as used herein, "and/or" refers to and encompasses any and all possible combinations of one or more of the listed items, as well as the lack of combinations when interpreted in the alternative ("or").

As used herein, "at least one" means "one or more" of the listed elements.

The term "alkyl" refers to a straight or branched hydrocarbon chain radical consisting solely of carbon and hydrogen atoms, containing no unsaturation, having from one to eight carbon atoms, and which is attached to the rest of the molecule by a single bond, such as illustratively, methyl, ethyl, n-propyl 1-methylethyl (isopropyl), n-butyl, n-pentyl, and 1,1-dimethylethyl (tert-butyl).

The term "cycloalkyl" denotes a non-aromatic mono or multicyclic ring system of 3 to 12 carbon atoms such as cyclopropyl, cyclobutyl, cyclopentyl, cyclohexyl and examples of multicyclic cycloalkyl groups include perhydronapththyl, adamantyl and norbornyl groups bridged cyclic group or spirobicyclic groups e.g., spiro(4,4)non-2-yl.

The term "leaving group," or "LG", as used herein, refers to any group that leaves in the course of a chemical reaction involving the group and includes but is not limited to halogen, brosylate, mesylate, tosylate, triflate, p-nitrobenzoate, phosphonate groups, for example.

The term "effective amount", as used herein, means any amount or dosage strength of the compound of the present invention to treat, minimize and/or prevent traumatic brain injury, including severe, moderate and/or mild TBI, including concussions. Effective amount, as used herein, also means any amount or dosage amount considered by the U.S. Food and Drug Administration (FDA) or other governmental agency or tribunal as being effective to treat, minimize and/or prevent traumatic brain injury, including severe, moderate and/or mild TBI, including concussions. Singular word forms are intended to include plural word forms and are likewise used herein interchangeably where appropriate and fall within each meaning, unless expressly stated otherwise.

Except where noted otherwise, capitalized and non-capitalized forms of all terms fall within each meaning.

Unless otherwise indicated, it is to be understood that all numbers expressing quantities, ratios, and numerical properties of ingredients, reaction conditions, and so forth used in the specification and claims are contemplated to be able to be modified in all instances by the term "about".

All parts, percentages, ratios, etc. herein are by weight unless indicated otherwise.

It should be further understood that the terms "TBI", "MTBI" and "concussion" as used herein, have the meanings set forth herein above.

### General Preparative Methods

In accordance with the present invention, the following general preparative methods for synthesizing the compound of the present invention are described with more detail in the reaction schemes/pathways and Examples presented below.

In accordance with certain synthetic transformations that may be employed in the synthesis of the compound of the present invention and in the synthesis of certain intermediates involved in the synthesis of the compound of the present invention, see for example, J. March. Advanced Organic Chemistry, 4th ed.; John Wiley: New York (1992); R.C. Larock. Comprehensive Organic Transformations, 2nd ed.; Wiley-VCH: New York (1999); F.A. Carey; R.J. Sundberg. Advanced Organic Chemistry, 2nd ed.; Plenum Press: New York (1984); T.W. Greene; P.G.M. Wuts. Protective Groups in Organic Synthesis, 3rd ed.; John Wiley: New York (1999); L.S. Hegedus. Transition Metals in the Synthesis of Complex Organic Molecules, 2nd ed.; University Science Books: Mill Valley, CA (1994); L.A. Paquette, Ed. The Encyclopedia of Reagents for Organic Synthesis; John Wiley: New York (1994); A.R. Katritzky; O. Meth-Cohn; C.W. Rees, Eds. Comprehensive Organic Functional Group Transformations; Pergamon Press: Oxford, UK (1995); G. Wilkinson; F.G A. Stone; E.W. Abel, Eds. Comprehensive Organometallic Chemistry; Pergamon Press: Oxford, UK (1982); B.M. Trost; I. Fleming. Comprehensive Organic Synthesis; Pergamon Press: Oxford, UK (1991); A.R. Katritzky; C.W. Rees Eds. Comprehensive Heterocylic Chemistry; Pergamon Press: Oxford, UK (1984); A.R. Katritzky; C.W. Rees; E.F.V. Scriven, Eds. Comprehensive Heterocylic Chemistry II; Pergamon Press: Oxford, UK (1996); and C. Hansch; P.G. Sammes; J.B. Taylor, Eds. Comprehensive Medicinal Chemistry: Pergamon Press: Oxford, UK (1990).

In addition, recurring reviews of synthetic methodology and related topics include Organic Reactions; John Wiley: New York; Organic Syntheses; John Wiley: New York; Reagents for Organic Synthesis: John Wiley: New York; The Total Synthesis of Natural Products; John Wiley: New York; The Organic Chemistry of Drug Synthesis; John Wiley: New York; Annual Reports in Organic Synthesis; Academic Press: San Diego CA; and Methoden der Organischen Chemie (Houben-Weyl); Thieme: Stuttgart, Germany. Furthermore, databases of synthetic transformations include Chemical Abstracts and which may be searched using either CAS OnLine or SciFinder, Handbuch der Organischen Chemie (Beilstein), and which may be searched using SpotFire, and REACCS.

The compound of the present invention is Compound J:

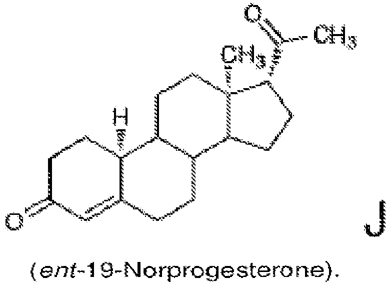

In accordance with the present invention, the compound of the invention of formula J is believed to be useful for treating, minimizing and/or preventing neuronal damage, such as neuronal damage, resulting from various injuries involving the brain, such as traumatic brain injury (TBI), whether the TBI is mild including concussions, moderate or severe traumatic brain injury.

Preferably, the compound of the invention is believed to be useful to treat and/or prevent MTBI. In another embodiment, the compound of the invention is believed to be useful to treat and/or prevent concussions.

The compound ent-19 norprogesterone, may be adminsitered in a dosage range of from about 0.05 mg/kg to 16 mg/kg, preferably from about 0.05 mg/kg to about 4 mg/kg and even more preferably from about 0.16 mg/kg to about 0.65 mg/kg or from about 1.13 mg/kg to about 45.2 mg/kg per 70 kg patient to treat, minimize and/or prevent TBI, including severe TBI, moderate TBI, mild TBI and concussions, prefreably mild TBI, and even more preferably concussions. While the higher dosage ranges are preferred, it nevertheless should be understood that any effective amount, as used herein, to treat, minimize and/or prevent TBI, including severe TBI, moderate TBI, mild TBI and concussions, prefreably mild TBI, and even more preferably concussions, is contemplated by the present invention. It is further contemplated that the compound of the present invention can be administered through a number of routes of administration that include, e.g., oral, sublingual, intravenous, intraperitoneal, subcutaneous, intramuscular, intraabdominal, ocular, otic, intranasal, topical, transdermal, subcutaneous and rectal routes of administration.

The present invention further contemplates that in some embodiments, the compound of the invention can be formulated into, e.g., compositions or admixtures and administered in a dosage form selected from, e.g., a tablet, capsule, gelcap, caplet, powder, granule, liquid, solution, suspension, dispersion, pellet, bead, eyedrop, gel, cream, ointment, salve, balm, lotion or suppository. In other embodiments, it is contemplated that the compound of the invention may be administered as a formulation that is swallowed, injected, infused, inhaled, applied transdermally or topically, such as applied to the skin, eyes, ears, nose, lungs, mucosal membranes or any other membrane, or inserted into the rectum. Nonetheless, it should be understood by those versed in the art that preferred routes of administration to treat and/or prevent TBI, especially, mild TBI and concussions, as contemplated by the present invention, is the topical, e.g., pernasal or inhalation, or injection route of administration. In one embodiment, the present invention provides the compound of the invention for administration through a route selected from oral, sublingual, intravenous, intraperitoneal, ocular, intranasal, transdermal, subcutaneous, and rectal. In another embodiment, the compound of the invention is administered orally. In another embodiment, the compound of the invention is administered sublingually. In another embodiment, the compound of the invention is administered by injection such as intravenously, intramuscularly, subcutaneously, or intraperitoneally. In another embodiment, the C-20 steroid compounds of Formulas I-IV are administered ocularly or otically. In another embodiment, the compound of the invention is administered intranasally. In another embodiment, the compound of the invention is administered transdermally. In another embodiment, the compound of the invention is administered subcutaneously. In another embodiment, the compound of the invention is administered rectally. In another embodiment, the compound of the invention is administered topically, including by inhalation.

In one embodiment, the compound of the invention is administered in a formulation selected from a tablet, capsule, gelcap, caplet, powder, solution, suspension, eyedrop, cream, ointment, lotion, gel or suppository. One of ordinary skill in the art will recognize that formulations that contain the compound of the invention as active agent, may optionally contain co-therapeutic agents and inactive excipients. In addition one of ordinary skill in the art will recognize that liquid formulations contain a solvent and that said solvent may be either aqueous or organic.

In one embodiment, the the compound of the invention is administered as a formulation that is swallowed, injected, infused, inhaled, applied topically such as to the skin, eye, mucosal or other membranes and lungs, or inserted into the rectum. One of ordinary skill in the art will recognize that some formulations are intended for specific routes of administration while other formulations can be used in multiple routes of administration. For example, solution formulations may be injected, infused, deposited intraperitoneally, deposited subcutaneously, applied to the eye, sprayed or applied into the nose or inhaled as a nebulized liquid or suspension. Alternatively, tablets, capsules, gelcaps and caplets are intended to be swallowed.

Additionally, suppositories are intended for insertion into the rectum while creams, ointments and lotions are intended for topical applications.

The inventive methods of the present invention to make the compound of the invention are illustrated in Schemes 1-15. In certain instances, reagents and solvents are listed. These reagents and solvents are exemplary and are not meant to be limited to the specific reagents or solvents shown.

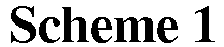

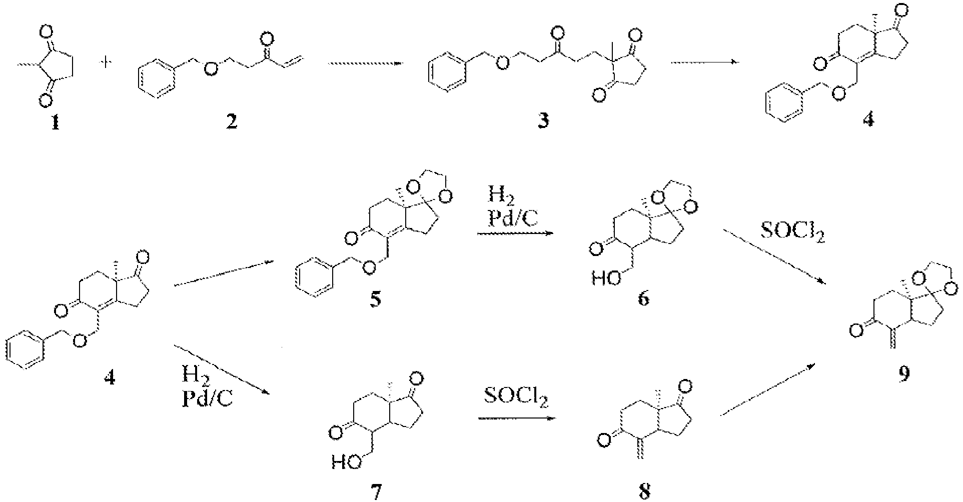

Scheme 1 represents the formation of compound (9) via two alternative processes. In Scheme 1, (1) is reacted with (2) to produce (3). The preparation of compound (2) is described in Yamauchi, Noriaki; Natsubori, Yoshiaki; Murae, Tatsushi Bulletin of the Chemical Society of Japan (2000), 73(11), 2513-2519). (3) is subjected to a stereoselective ring closing to form (4). Then (4) can be converted to (9) either: by selective protection of the carbonyl group to form (5) (as described in Bosch, M.P.; Camps, F.; Coll, J.; Guerrero, T.; Tatsuoka, T.; Meinwald, J. J. Org. Chem. 1986, 51, 773) followed by simultaneous hydrogenation of the ring double bond and cleavage of the benzyl ether to form (6) and elimination of the hydroxyl group therein with thionyl chloride; or by simultaneous hydrogenation of the ring double bond and cleavage of the benzyl ether to form (7) followed by elimination of the hydroxyl group therein with thionyl chloride to form (8) and protection of the carbonyl group (as described in Bosch, M.P.; Camps, F.; Coll, J.; Guerrero, T.; Tatsuoka, T.; Meinwald, J. J. Org. Chem. 1986, 51, 773).

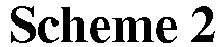

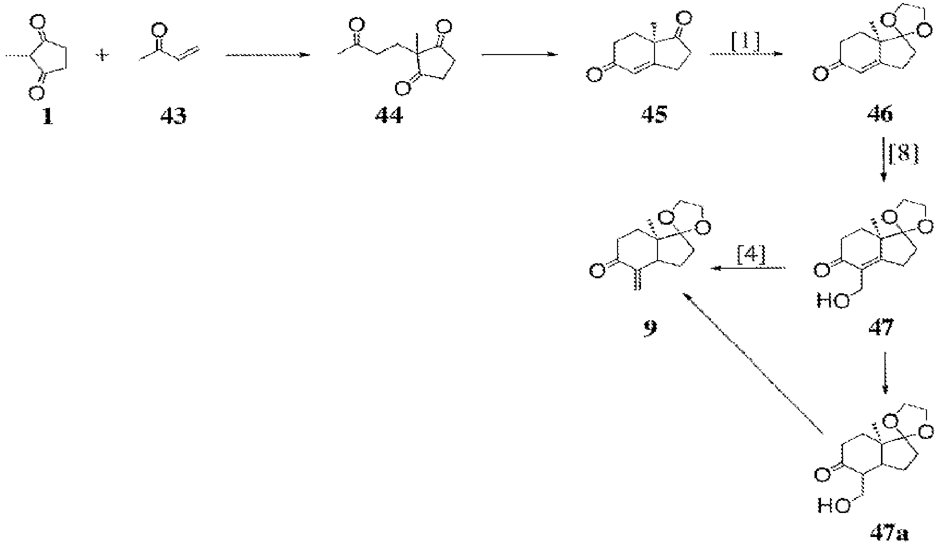

Scheme 2 represents an alternative to the formation of compound (9) of Scheme 1 from the combination of (1) and but-3-en-2-one (43). (1) and (43) are reacted to form (44) which is subjected to a stereoselective ring closing reaction to form (45). (45) is then selectively protected to form (46) (Bosch, M.P.; Camps, F.; Coll, J.; Guerrero, T.; Tatsuoka, T.; Meinwald, J. J. Org. Chem. 1986, 51, 773) which is subjected to a Baylis-Hillman reaction to form (47) (Satyanarayana reaction (Basavaiah, D.; Rao, A. J.; Satyanarayana, T. Chem. Rev. 2003, 103, 811). (47) is subjected to a Lewis acid facilitated reduction resulting in compound (9) of Scheme 1. Alternatively, (47) is hydrogenated giving (47a). Subsequent activation of the alcohol and elimination results in compound (9) of Scheme 1.

In certain embodiments, the conversion of (47a) to (9), and similar reactions, may utilize A1203 as a reagent.

One of ordinary skill in the art will recognize that activation of a beta-hydroxyketone and subsequent elimination reactions such as those described in Scheme 2 may be accomplished under a variety of conditions including, but not limited to KOH, methanesulfonyl chloride with diisopropylethylamine, para-toluenesulfonyl chloride with dimethylaminopyridine, DCC, pyridinium hydrochloride, alumina.

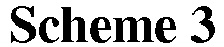

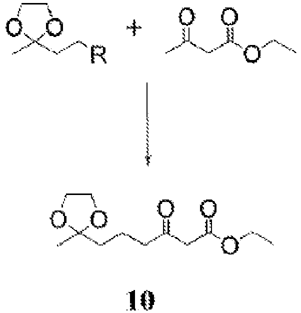

Scheme 3 represents a one step process to form compound (10) by reaction of substituted 2-ethyl-2-methyl-1,3-dioxolane a with ethyl 3-oxobutanoate. In certain embodiments, and without being limited thereto, leaving group R is -OTs, -OMs, -OTf, -CI, -Br, or -I. In still other embodiments, leaving group R is -OTs, -Br, or -I. In yet other embodiments, leaving group R is -Br.

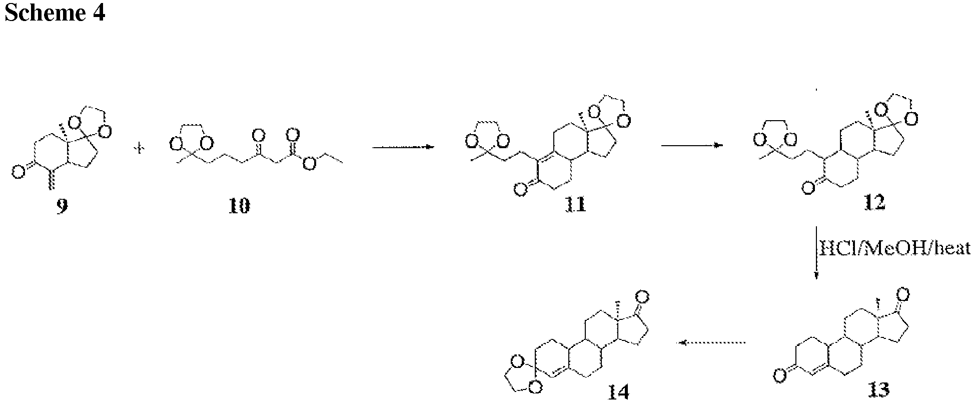

Scheme 4 represents the formation of compound (14) from the combination of (9) and (10). In Scheme 4, (9) and (10) are reacted to form (11) which is hydrogenated to form (12). (12) is then double deprotected and cyclized to form (13) which is selectively reprotected to form (14) (Tsunoda, T.; Suzuki, M.; Noyori, R. Tetrahedron Lett. 1980, 21, 1357).

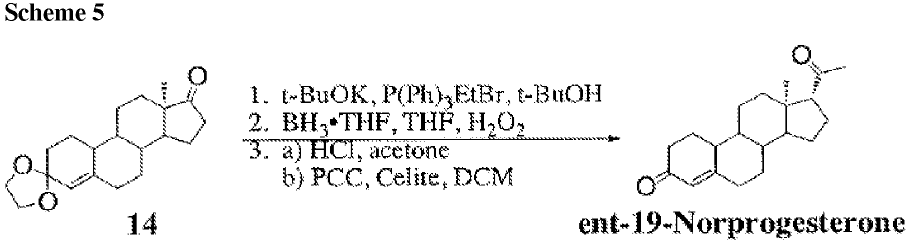

Scheme 5 represents the formation of ent-19-Norprogesterone from compound (14) of Scheme 4. In Scheme 5, (14) is reacted with potassium tert-butoxide and ethyl triphenylphosphonium bromide followed by hydroboration and oxidation to form ent-Progesterone. One of ordinary skill in the art will recognize that hydrolysis of the ketal protecting group can be done either before oxidation or after oxidation. One of ordinary skill in the art will further recognize that there are many reaction conditions and reagents suitable for the oxidation of an alcohol to a ketone and that alternatives to PCC include, but are not limited to, Swern, KMnO4, Dess-Martin, TEMPO and IBX.

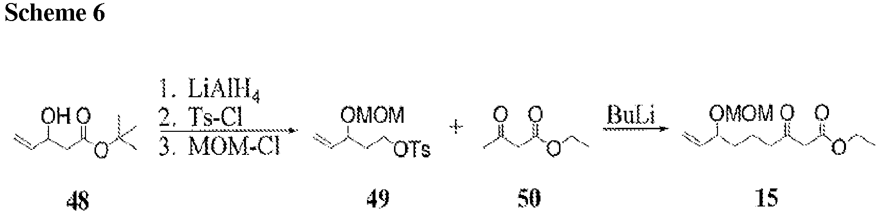

Scheme 6 represents the formation of compound (15) from the tert-butyl 3-hydroxypent-4-enoate (48) via reduction (Batt, Frederic and Fache, Fabienne, European Journal of Organic Chemistry, 2011(30), 6039-6055, S6039/1-S6039/46; 2011), formation of a tosylate and protection with a MOM (Methoxymethyl ether) protecting group to form (49). (49) is then reacted with ethyl 3-oxobutanoate (50) in the presence of a base to form (15).

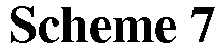

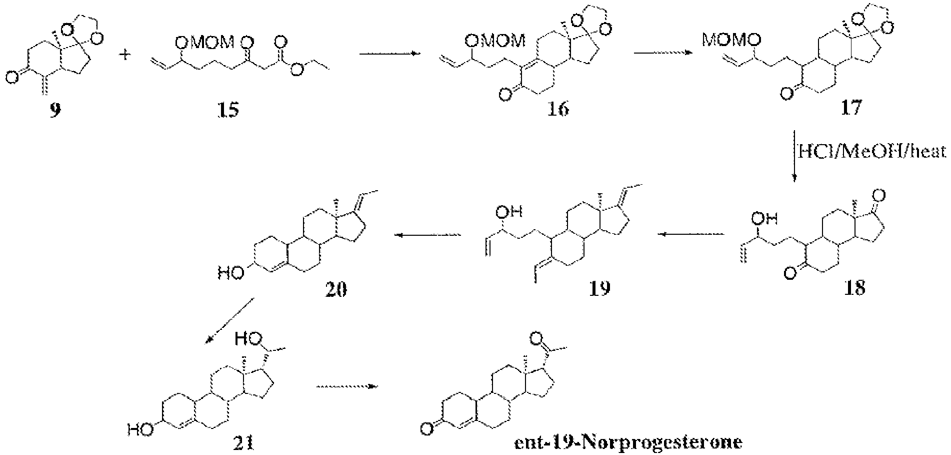

Scheme 7 represents the formation of ent-19-Norprogesterone from the combination of (9) from Scheme 1 and (15) from Scheme 6. In Scheme 7, (9) and (15) are reacted in a Robinson annulation to form (16) which is subjected to a Birch reduction or selective hydrogenation reaction to form (17). The MOM ether and ketal of (17) are simultaneously removed to form (18) which is then subjected to a double Wittig reaction to form (19). (19) then undergoes a ring closing metasthesis reaction to form (20) which is subjected to hydroboration reaction to form (21). Double oxidation of (21) results in formation of ent-19-Norprogesterone.

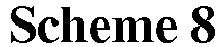

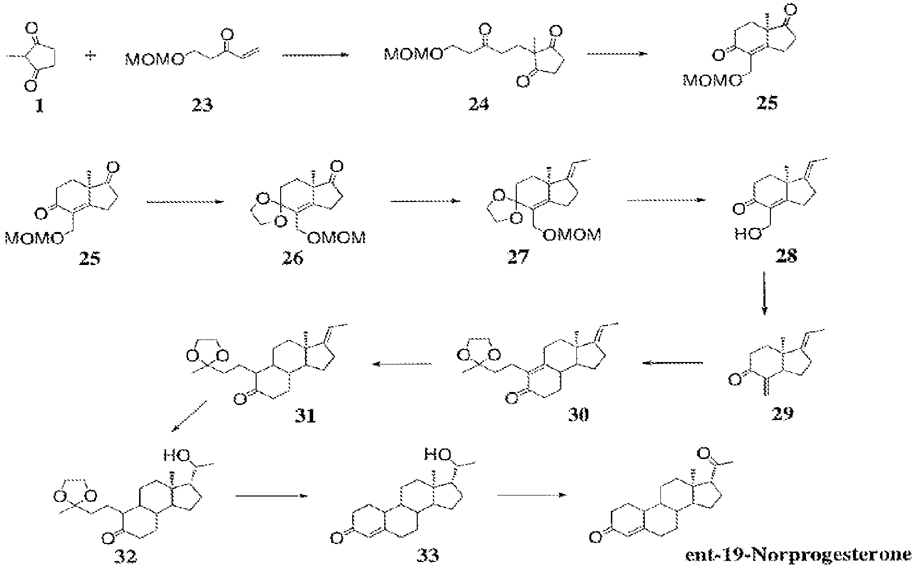

Scheme 8 represents the formation of ent-19-Norprogesterone from the combination of (1) from Scheme 1 with a methoxymethylether protected compound (23). (1) and (23) are reacted to form (24) which is subjected to a stereoselective cyclization reaction to form (25). (25) is then selectively protected to form (26) (Tsunoda, T.; Suzuki, M.; Noyori, R. Tetrahedron Lett. 1980, 21, 1357) which is subjected to a Wittig reaction with ethyl triphenylphosphonium bromide to form (27). The MOM ether and the ketal of (27) are simultaneously hydrolyzed to form (28) which is then subjected to a Lewis acid facilitated reduction to form the exocyclic double bond in (29) (Das, Biswanath; Banerjee, Joydeep; Chowdhury, Nikhil; Majhi, Anjoy; Holla, Harish, Synlett (2006), (12), 1879-1882). (29) is subjected to a Robinson annulation with (10) from Scheme 3 to form (30) which is subjected to a Birch reduction or selective hydrogenation to form (31). (31) undergoes a hydroboration reaction to form (32). Hydrolysis of the ketal of (32) with tandem aldol cyclization forms (33). Oxidation of (33) results in ent-19-Norprogesterone.

In certain embodiments, the Lewis acid facilitated reduction is replaced by a hydrogenation and beta-elimination 2-step sequence.

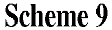

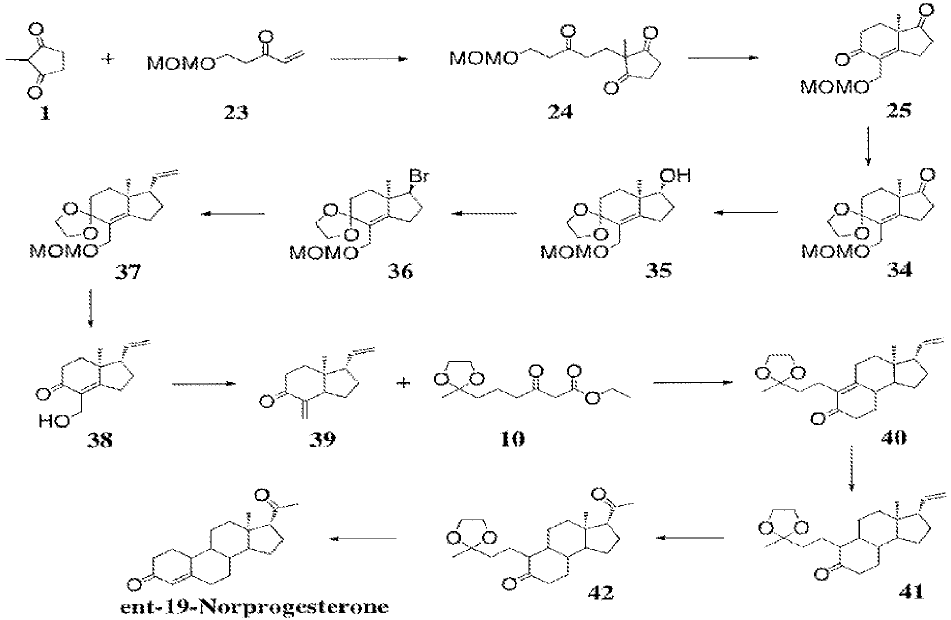

Scheme 9 represents an alternative to formation of ent-19-Norprogesterone from Scheme 8. As illustrated, compound (25) is prepared as described in Scheme 8. Continuing, compound (25) is selectively protected to produce the acetal compound (34) (Tsunoda, T.; Suzuki, M.; Noyori, R. Tetrahedron Lett. 1980, 21, 1357) which is stereoselectively reduced to form the hydroxyl compound (35). (35) is brominated with inversion of stereochemistry to form (36) which is subjected to a nucleophilic displacement with a vinyl anion and inversion of stereochemistry to form (37). The MOM ether and ketal of (37) are simultaneously hydrolyzed to form (38) which is then subjected to Lewis acid facilitated reduction to form the exocyclic double bond in (39) (Das, Biswanath; Banerjee, Joydeep; Chowdhury, Nikhil; Majhi, Anjoy; Holla, Harish, Synlett (2006), (12), 1879-1882). (39) is reacted with compound (10) formed in Scheme 3 via a Robinson annulation to form (40) which is subjected to a Birch reduction or selective hydrogenation to form (41). (41) undergoes a Wacker oxidation to form (42). Tandem ketal hydrolysis and aldol cyclization of (42) results in ent-19-Norprogesterone.

In certain embodiments, the Lewis acid facilitated reduction is replaced by a hydrogenation and beta-elimination 2-step sequence.

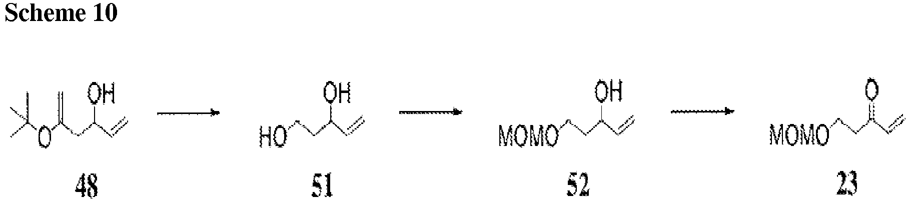

Scheme 10 represents the preparation of compound (23) illustrated in Scheme 9. This chemistry is adapted from a protocol for the preparation of a related compound (Batt, F.; Fache, F. Eur. J. Org. Chem. 2011, 6039). As illustrated, compound (48) is reduced to compound (50) (Scheme 6). The primary hydroxyl group of compound (51) (Batt, F.; Fache, F. Eur. J. Org. Chem. 2011, 6039) is then selectively converted to the corresponding methoxymethyl ether (52). Compound (52) is then oxidized to form compound (23).

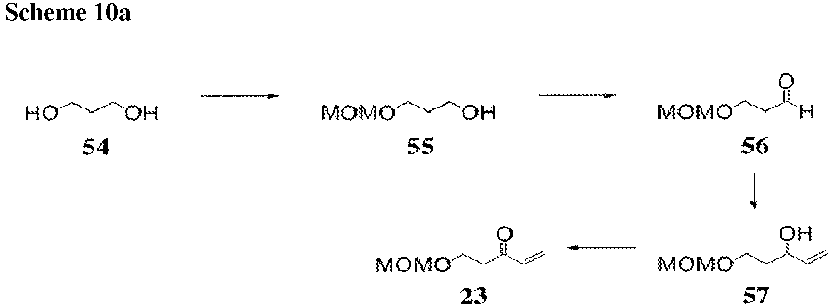

Scheme 10a represents an alternative to the preparation of compound (23) illustrated in Scheme 10. This chemistry is adapted from a protocol for the preparation of a related compound (Batt, F.; Fache, F. Eur. J. Org. Chem. 2011, 6039). As illustrated, propylene glycol is converted to its mono-methoxymethyl ether compound (55). The free hydroxyl group is then oxidized to form the aldehyde of compound (56). The aldehyde is then converted to the allylic alcohol compound (57). Compound (57) is then oxidized to form compound (23).

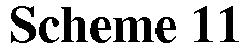

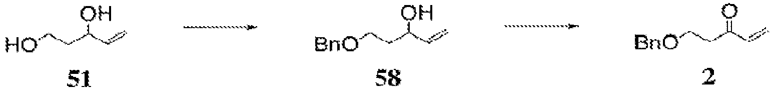

Scheme 11 represents the preparation of compound (2) illustrated in Scheme 1. This chemistry is adapted from a protocol for the preparation of a related compound (Batt, F.; Fache, F. Eur. J. Org. Chem. 2011, 6039) and represents an alternative to the synthesis described in Yamauchi, Noriaki; Natsubori, Yoshiaki; Murae, Tatsushi Bulletin of the Chemical Society of Japan (2000), 73(11), 2513-2519). As illustrated, the primary hydroxyl group of compound (51) (Batt, F.; Fache, F. Eur. J. Org. Chem. 2011, 6039) is selectively converted to the corresponding benzyl ether (58). Compound (58) is then oxidized to form compound (2).

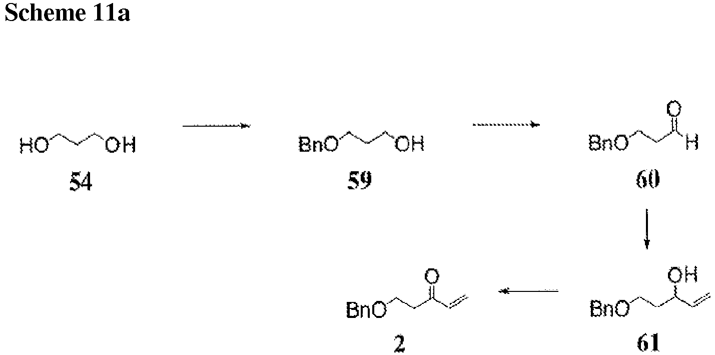

Scheme 11a represents an alternative to the preparation of compound (2) illustrated in Scheme 11. This chemistry is adapted from a protocol for the preparation of a related compound (Batt, F.; Fache, F. Eur. J. Org. Chem. 2011, 6039) and represents an alternative to the synthesis described in Yamauchi, Noriaki; Natsubori, Yoshiaki; Murae, Tatsushi Bulletin of the Chemical Society of Japan (2000), 73(11), 2513-2519). As illustrated, propylene glycol is converted to its mono-benzyl ether compound (59). The free hydroxyl group is then oxidized to form the aldehyde of compound (60). The aldehyde is then converted to the allylic alcohol compound (61). Compound (61) is then oxidized to form compound (2).

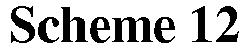

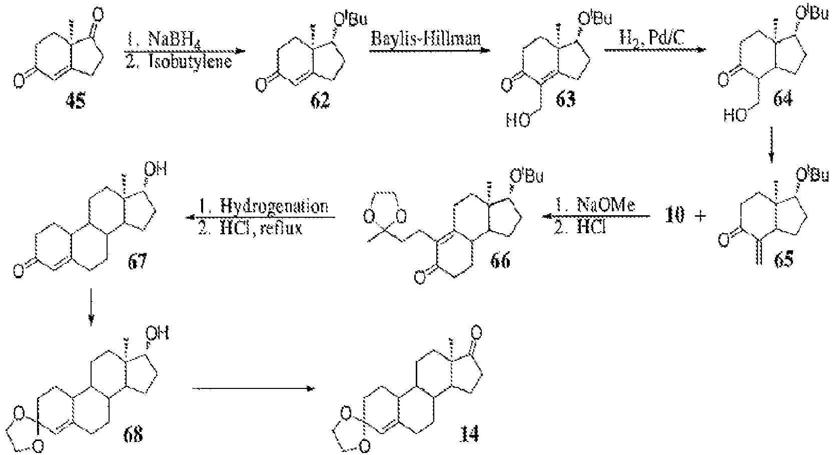

Scheme 12 provides an alternative synthesis of Compound (14) as described in Scheme 4. The synthesis includes the sequence converting compound (62) to compound (65) and the conversion of ent-19-nortestosterone (compound 67) to the dioxolane ketal compound (68).

Specifically, (45) is reduced and protected to form (62). (62) is subject to a Baylis-Hillman reaction to form (63) which is further reduced to form (64). (64) is subject to an elimination reaction to form the double bond in (65). (65) is reacted with Compound (10) from Scheme 3 to form (66) which is hydrogenated and cyclized to form ent-19-nortestosterone (67). ent-19-nortestosterone (67) is then ketal protected and reduced to form (14).

In certain embodiments, the conversion of compound (63) to compound (65) is accomplished in a single step comprising a Lewis acid facilitated reduction.

One of ordinary skill in the art will recognize that activation of a beta-hydroxyketone and subsequent elimination reactions such as those described in Scheme 12 may be accomplished under a variety of conditions including, but not limited to KOH, methanesulfonyl chloride with diisopropylethylamine, para-toluenesulfonyl chloride with dimethylaminopyridine, DCC, pyridinium hydrochloride, alumina.

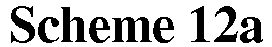

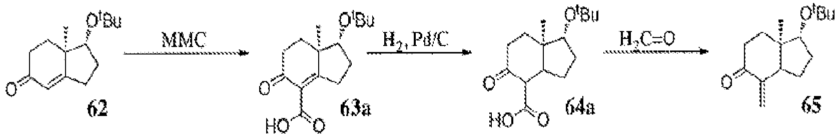

Scheme 12a provides an alternative conversion of compound (62) to compound (65). As illustrated, compound (62) is treated with methyl magnesium carbonate (MMC) forming the carboxylated product compound (63a). Catalytic hydrogenation reduces the olefin of compound (63a) forming compound (64a). Final decarboxylation in the presence of formaldehyde forms compound (65). In some embodiments, the conversion of compound (63a) to compound (64a) and the conversion of compound (64a) to compound (65) are distinct and separate synthetic steps. In other embodiments, the conversion of compound (63a) to compound (64a) and the conversion of compound (64a) to compound (65) are run in tandem. One of ordinary skill in the art will recognize that there are many catalysts useful for the reduction of a double bond to a single bond including, but not limited to, palladium on carbon, platinum on carbon, palladium hydroxide on carbon, palladium, platinum and Raney nickel.

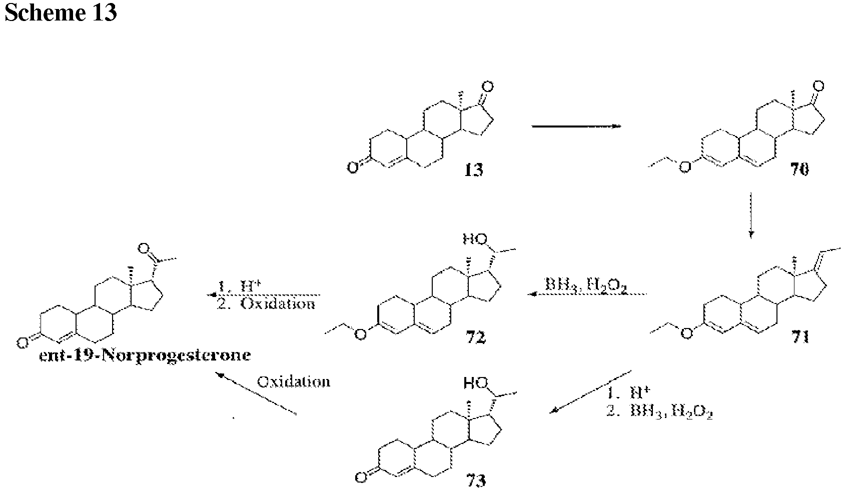

Scheme 13 represents an alternative continuation from compound (13) (Scheme 4) and depends upon the conversion of (13) to the ethyl enol ether compound (70) followed by the Wittig reaction generating compound (71). Reactions of this type are generally described by Antimo, et al., [Steroids 77 (2012) 250-254]. This sequence is completed by initial borane oxidation of (71) followed by hydrolysis of the enol ether and oxidation to form (72). Alternatively, (71) is initially hydrolyzed followed by borane oxidation giving compound (73).

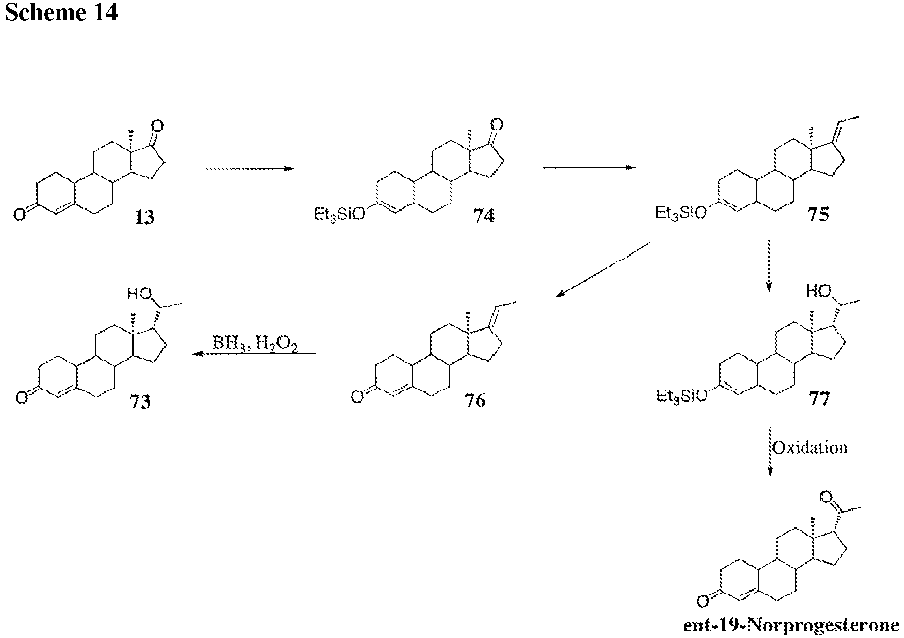

Scheme 14 represents an alternative to Scheme 13 and utilizes a reductive silylation to protect the enone of (13) to form (74). Protection of this type is generally described in Iwao, et al. [Tetrahedron Letters 49 (1972) 5085-5038] and Horiguchi, et al. [Journal of the American Chemical Society 111(16) (1989) 6259-6265]. Following borane oxidation of (75) to (77), oxidation of the alcohol and oxidative deprotection of the enone generates ent-19-Norprogesterone. Deprotection of this type is generally described by Yoshihiko, et al. [Journal of Organic Chemistry 43(5) (1978) 1011-1013].

Alternatively, the silyl enol ether (75) is initially oxidatively converted to (76) followed by borane oxidation to compound (73).

### Examples

### Abbreviations and Acronyms

A comprehensive list of the abbreviations used by organic chemists of ordinary skill in the art appears in The ACS Style Guide (third edition) or the Guidelines for Authors for the Journal of Organic Chemistry. The abbreviations contained in said lists, and all abbreviations utilized by organic chemists of ordinary skill in the art are hereby incorporated by reference. For purposes of this invention, the chemical elements are identified in accordance with the Periodic Table of the Elements, CAS version, Handbook of Chemistry and Physics, 67th Ed., 1986-1987.

More specifically, when the following abbreviations are used throughout this disclosure, they have the following meanings:
- atm: atmosphere
- br s: broad singlet
- Buchi: rotary evaporator ®BUCHI Labortechnik AG
- C: Celsius
- CDCI₃: deuterated trichloromethane
- Celite: diatomaceous earth filter agent ®Celite Corp.
- d: doublet
- dd: doublet of doublets
- DIBAL-H: diisobutylaluminum hydride
- DCM: dichloromethane
- DMI: dimethyl-2-imidazolidinone
- g: gram
- h: hour, hours
- ₁H NMR: proton nuclear magnetic resonance
- HPLC: high performance liquid chromatography
- *J*: coupling constant (NMR spectroscopy)
- L: liter
- LAH: lithium aluminum hydride
- LG: leaving group
- M: mol L-1 (molar)
- m: multiplet
- MHz: megahertz
- min: minute, minutes
- mL: milliliter
- pM: micromolar
- mol: mole
- MS: mass spectrum, mass spectrometry
- m/z: mass-to-charge ratio
- N: equivalents L-1 (normal)
- NBS: N-bromo succinimide
- NMO: N-Methylmorpholine-N-Oxide
- NMR: Nuclear Magentic Resonance
- pH: negative logarithm of hydrogen ion concentration
- q: quartet
- RBF: round bottom flask
- r.t: room temperature
- RT: retention time (HPLC)
- rt: room temperature
- s: singlet
- t: triplet
- THE: tetrahydrofuran
- TLC: thin layer chromatography
- TsCI: tosyl chloride

The percentage yields reported in the following examples are based on the starting component that was used in the lowest molar amount. Air and moisture sensitive liquids and solutions are transferred via syringe or cannula, and are introduced into reaction vessels through rubber septa. Commercial grade reagents and solvents are used without further purification. The term "concentrated under reduced pressure" refers to use of a Buchi rotary evaporator or equivalent equipment at approximately 7332.73 Pa (55 mm Hg).

All temperatures are reported uncorrected in degrees Celsius (°C). Thin layer chromatography (TLC) is performed on pre-coated glass-backed silica gel 60 A F¬254 250 pm plates.

The structures of compounds of this invention are confirmed using one or more of the following procedures.

### NMR

NMR spectra are acquired for each compound when indicated in the procedures below. NMR spectra obtained were consistent with the structures shown. Routine one-dimensional NMR spectroscopy was performed on a 300 MHz Brucker spectrometer. The samples were dissolved in deuterated solvents. Chemical shifts were recorded on the ppm scale and were referenced to the appropriate solvent signals, such as 2.49 ppm for DMSO-d6, 1.93 ppm for CD3CN, 3.30 ppm for CD3OD, 5.32 ppm for CD2C12 and 7.26 ppm for CDCI3 for 1H spectra.

### Materials

Equipment used in the execution of the chemistry of this invention include but is not limited to the following:
- Low temperature vacuum pump - Zhengzhouchangcheng Experimental Equipment Co., Ltd (Model # DLSB-10/20)
- Rotary evaporator - Shanghaizhenjie Experimental Equipment Co., Ltd (Model # RE-52CS)
- Oil pump - Shanghai Vacuum pump factory (Model # 2XZ-4)
- Mechanical stirrer - Beijingshijiyuhua Experimental Equipment Co., Ltd (Model # DW-3-300)
- Vacuum drying oven - Beijinglianhekeyi Experimental Equipment Co., Ltd (Model # DZF-6020)
- LCMS - Agilent (Model # 1200-6100)
- GCMS - Agilent (Model # 7890A-5975C)
- GC - Agilent (Model # 7890A)
- Chiral HPLC - Shimadzu (Model # LC-20AT)
- NMR - Bruker (Model # AVANCEB1300)
- Liquid chromatograph - Agilent (Model # G1322A)
- High temperature oil bath - SMS (Model # 00508)
- Electronic balance - LBTEC (Model # XS205DU)

Chemicals and solvents that are used in the experimental workups are purchased from either Sigma Aldrich, Fisher Scientific or EMD unless otherwise stated and the solvents used are either ACS or HPLC grade with the two grades being used interchangeably. For TLC analysis, the silica 60 gel glass backed TLC plates are used.

### EXAMPLE 1 - Preparation of compound 3 (Scheme 1)

2-Methyl-1,3-pentanedione (1 g, 1.2 eq.) is dissolved in anhydrous acetonitrile (40 mL) and 5-benzyloxy-pent-1-ene-2-one (1.5 g, 1.0 eq.) is added followed by triethylamine (50 mg, 0.05 eq.). The reaction is stirred at 25-30 deg C for 12 hours after which, it is concentrated to dryness. Purification of the residue on silica gel (Ethyl acetate/Hexane 1/5) gives compound 3 (1.8 g) as a colorless oil. 1H NMR (300 MHz, CDCI3): 61.10 (s, 3H), 1.90 (t, 2H), 2.50 (t, 2H), 2.65 (t, 2H), 2.70-2.90 (m, 4H), 3/0 (t, 2H), 4.50 (s, 2H), 7.25-7.4 (m, 5H). MS (M+ + 1) 303.1.

### EXAMPLE 2 - Preparation of compound 46 (Scheme 2)

2-Ethyl-2-methyl-1,3-dioxolane (120mL) and compound 45 (20 g, 1.0 eq.) are combined under nitrogen. Ethylene glycol (1.2 mL, 0.14 eq.) is added followed by p-toluenesulfonic acid (390 mg, 0.02 eq.). The reaction is stirred at 25-30 deg C for 96 hours until the concentration of compound 45 is less than 20% as measured by HPLC. Ethyl acetate (100 mL) is added and the resulting mixture is washed with water (2 x 100 mL), is dried over anhydrous sodium sulfate, is filtered and is concentrated to dryness. The residue is purified on silica gel (ethyl acetate/hexane 1/20) yielding compound 46 (8 g) as a colorless oil. 1H NMR (300 MHz, CDCI3): 6 1.20-1.35 (m, 7H), 1.60-1.70 (m, 1H), 1.90-2.00 (m, 1H), 2.10-2.80 (m, 6H), 3.85-4.05 (m, 4H), 5.85 (s, 1H). MS (M+ + 1) 209.1.

### EXAMPLE 3 - Preparation of compound 47 (Scheme 2)

Compound 46 (8.0 g, 1.0 eq.) is added to a mixture of 1,4-dioxane (40 ml) and water (34 mL). Formaldehyde (3.1 g, 1.0 eq.) is then added followed by 1,4-diazabicyclo[2.2.2]octane (DABCO, 8.5 g, 1.0 eq). The reaction is stirred at 25-30 deg C for 120 hours after which, ethyl acetate (100 mL) is added. The mixture is washed with water (2 x 100 mL), is dried over anhydrous sodium sulfate, is filtered and is concentrated to dryness. Purification of the residue on silica gel (10% ethyl acetate in hexane) gives compound 47 (5 g) as a colorless oil. 1H NMR (300 MHz, CDCI3): 6 1.25 (m), 1.65 (m, 1H), 1.95 (m, 1H), 2.15-2.80 (m), 3.90-4.05 (m), 5.80 (s, 1H).

### EXAMPLE 4 - Preparation of compound 47a (Scheme 2)

Compound 47 (2 g) is dissolved in anhydrous tetrahydrofuran (THF, 200 mL) under a nitrogen atmosphere. 10% Pd/C (200 mg) is added and the reaction is placed under a hydrogen atmosphere. The reaction is stirred at -10-0 deg C over 40 hours after which, the Pd/C is removed by filtration. The filtrate is concentrated to dryness and the residue is purified on silica gel (10% ethyl acetate/hexane) giving compound 47a (1.6 g) as a colorless oil. 1H NMR (300 MHz, DMSO-d6): 6 0.95-1.15 (m, 1H), 1.55-2.10 (m), 2.50 (t, 2H), 2.40-2.50 (m, 1H), 2.70-2.80 (q, 1H), 3.15-3.30 (m, 1H), 3.65-3.90 (m), 4.35 (dd, 1H). MS (M+ + 1) 241.1.

### EXAMPLE 5 - Preparation of compound 9 (Scheme 2)

Compound 47a (300 mg, 1.0 eq.) is dissolved in dichloromethane (DCM, 3 mL) and triethylamine (TEA, 3.0 eq.) is added. The mixture is cooled to - 10 deg C under nitrogen and methanesulfonyl chloride (1.2 eq.) is added dropwise. Stirring is continued at 10-20 deg C for 4 hours after which, toluene (3 mL) is added followed by 1,8-diazabicycloundec-7-ene (DBU, 3.0 eq.). Stirring is continued at 25-30 deg C for an additional 40 hours after which, the reaction is washed with water (2 x 3 mL), is dried over anhydrous sodium sulfate, is filtered and is concentrated to dryness. The residue is purified on silica gel (ethyl acetate/hexane 1/10) giving compound 9 (100 mg) as a colorless oil. 1H NMR (300 MHz, DMSO-d6): ö 1.00 (s, 3H), 1.40-1.60 (m, 2H), 1.70¬2.00 (m, 4H), 2.30-2.55 (m, 2H), 2.80 (m, 1H), 3.80-3.95 (m, 4H), 5.20 (s, 1H), 5.70 (s, 1H). MS (M+ + 1) 223.1.

### EXAMPLE 6 - Preparation of compound 10 (Scheme 3)

Sodium hydride (426 mg, 1.2 eq.) is placed under nitrogen and cooled to 0 deg C. Tetrahydrofuran (THF, 10 mL) is added followed by hexamethylphosphoramide (HMPA, 326 mg, 0.25 eq.). Ethyl acetoacetate (1 mL, 1.0 eq.) is added and the mixture is stirred at 0 deg C for 10 minutes. n-Butyllithium (2.5M, 3.6 mL, 1.1 eq.) was added and the mixture is stirred at 0 deg C for an additional 10 minutes. 2-(2-methyl-1,3-dioxolan-2-yl)ethylbromide (1.6 g, 1.0 eq.) is added and the reaction is stirred at 0 deg C for 30 minutes. The reaction is quenched with aqueous oxalic acid (10%, 20 mL) and is washed with dichloromethane (DCM, 3 x 20 mL). The organic phase is additionally washed with saturated aqueous sodium bicarbonate (30 mL) and brine (30 mL). The organic phase is dried over anhydrous sodium sulfate, is filtered and is concentrated. The residue is purified on silica gel (ethyl acetate/hexane 1/30) giving compound 10 (600 mg) as a yellow oil. 1H NMR (300 MHz, DMSO-d6): ö 1.25 (t, 3H), 1.30 (s, 3H), 1.60-1.80 (m, 4H), 2.60 (t, 2H), 3.45 (s, 2H), 3.90-4.00 (m, 4H), 4.15-4.25 (q, 2H).

### EXAMPLE 7 - Preparation of compound 11 (Scheme 4)

Compound 9 (500 mg, 1.0 eq.) is dissolved in methanol (15 mL) and compound 10 (715 mg, 1.3 eq.) is added. Sodium methoxide (0.2eq)is added and the mixture is stirred at 30 deg C for 16 hours. Aqueous sodium hydroxide (5 M, 5.0 eq.) is added and the reaction is stirred for an additional 4 hours at 30 deg C. The methanol is then removed utilizing a rotary evaporator. Water (5 mL) is then added and the mixture is washed with toluene (2 x 3 mL). The aqueous phase is cooled to 0 deg C and is acidified to pH 6 with aqueous HCI (6 N). The mixture is washed with ethyl acetate and the organic extract is concentrated to dryness. The residue is purified on silica gel (ethyl acetate/hexane 1/10) giving compound 11 (150 mg) as a colorless oil. MS (M+ + 1) 377.1.

### EXAMPLE 8 - Preparation of ent-19-Norprogesterone (Scheme 5)

### (a) Wittig Reaction

Ethyl triphenylphosphonium bromide (2.8 g, 3 equivalents) and potassium tert-butoxide (1.0 g, 3.0 equivalents) are combined in anhydrous tert-butanol (10 mL) under nitrogen. The mixture is heated to 75-80 deg C for 20 minutes after which, compound 14 (1.0 g, 1 equivalent) is added. The reaction is stirred at 75¬80 deg C for 3 hours after which, it is cooled to 20-25 deg C and is quenched with brine (20 mL). The resulting mixture is washed with ethyl acetate (3 x 20 mL). The combined organic extracts are dried over anhydrous sodium sulfate, are filtered and are concentrated to dryness. The residue is purified on silica gel (10% ethyl acetate/hexane) giving the desired Wittig product in 90% yield. MS (M+ + 1) 329.3

### (b) Borane Hydration

The Wittig product from part (a) (1.0 g, 1 equivalent) is placed under a nitrogen atmosphere and is dissolved in anhydrous tetrahydrofuran (THF, 100 mL). Borane-THF complex (1 M in THF, 3.0 mL, 1 equivalent) is added and the reaction is stirred at 20-25 deg C for 3 hours. The reaction is then concentrated to dryness and sodium hydroxide solution (10% in water, 50 mL) is added followed by hydrogen peroxide solution (30% in water, 0.5 mL). The resulting mixture is stirred at 20-25 deg C for an additional 1 hour after which, water (100 mL) is added. The mixture is then washed with dichloromethane (2 x 100 mL) and the combined organic extracts are washed with brine (50 mL). Concentration of the organic phase yields the crude alcohol which is used in the following step without purification.

### (c) Ketal Hydrolysis

The crude product from step (b) (2.0 g, 1 equivalent) is dissolved in acetone (20 mL) and hydrochloric acid (30% in water, 20 mL) is added. The reaction is stirred at 20-25 deg C for 30 minutes after which, it is concentrated to dryness. The residue is dissolved in ethyl acetate (50 mL) and water (30 mL) is added. After stirring vigorously for 5 minutes, the phases are separated and the organic phase is washed with saturated aqueous sodium bicarbonate (2 x 25 mL) and brine (25 mL). The organic phase is then concentrated to dryness and the residue is purified on silica gel (10% ethyl acetate/hexane) giving the desired enone in 45% overall yield from the Wittig product. 1H NMR (300 MHz, DMSO-d6): 65.70 (s, 1H), 4.15 (d, 1H), 3.40-3.50 (m, 1H), 2.40-2.45 (m, 1H), 2.10-2.35 (m, 5H), 1.70-1.85 (m, 4H), 1.50-1.60 (m, 2H), 1.40-1.50 (m, 1H), 1.25-1.35 (m, 1H), 1.15-1.25 (m, 2H), 0.90-1.15 (m, 7H), 1.85-1.95 (m, 1H), 0.65 (s, 3H). MS (M+ + 1) 303.2.

### (d) Oxidation to ent-19-Norprogesterone

Sodium acetate (1.20 g, 10 equivalents), pyridinium chlorochromate (PCC, 1.90 g, 4 equivalents), and the enone from step (c) (0.5 g, 1 equivalent) are combined with dichloromethane (50 mL) under nitrogen. The mixture is stirred at 20-25 deg C for 3 hours after which, it is filtered. The filter cake is washed with dichloromethane and the combined filtrates are concentrated to dryness. The residue is purified on silica gel (30% ethyl acetate/hexane) giving ent-19-norprogesterone in 90% yield. 1H NMR (300 MHz, DMSO-d6): 65.70 (s, 1H), 2.55-2.60 (t, 1H), 2.40-2.50 (m, 1H), 2.10-2.35 (m, 5H), 2.05 (s, 3H), 1.95-2.05 (m, 1H), 1.70-1.90 (m, 2H), 1.10-1.70 (m, 9H), 0.90-1.10 (m, 1H), 0.75-0.90 (m, 1 H), 0.60 (s, 3H). MS (M+ + 1) 301.1.

### EXAMPLE 9 - Preparation of compound 48 (Scheme 6)

Compound 48 is prepared as described by Batt, et al. (Eur. J. Org. Chem., 2011, 6039-6055).

### EXAMPLE 10 - Preparation of compound 49 (Scheme 6)

Compound 48 (100 g) is reduced to the corresponding alcohol using lithium aluminum hydride as described by Batt, et al. (Eur. J. Org. Chem., 2011, 6039-6055). The resulting diol (1 g, 1.0 eq.) is dissolved in dichloromethane (DCM, 10 mL) under nitrogen. Triethylamine (2.0 eq.) is added and the resulting mixture is cooled to 0 deg C. Para-toluenesulfonyl chloride (1.0 eq.) is added slowly and the reaction is stirred at 0 deg C for 30 minutes. The resulting mixture is washed with water (10 mL) after which, it is dried over anhydrous sodium sulfate, is filtered and is concentrated to dryness. The residue is purified on silica gel (ethyl acetate/hexane 1/10) giving the desired primary tosylate (500 mg) as a yellow oil. The resulting primary tosylate (100 mg, 1.0 eq.) is dissolved in DCM (10 mL) under nitrogen. Diisopropylethyl amine (DIEA, 1.2 eq.) is added and the mixture is cooled to 0 deg C. Methoxymethyl chloride (1.0 eq) is added dropwise and the reaction is stirred from 0-25 deg C over 2 hours after which, it is washed with water (10 mL). The organic phase is dried over anhydrous sodium sulfate, is filtered and is concentrated to dryness. The residue is purified on silica gel (Ethyl acetate/hexane 1/20) giving the desired compound 49 (60 mg) as a yellow oil.

### EXAMPLE 11 - Preparation of compound 24 (Scheme 9)

2-Methyl-1,3-cyclopentanedione (3.0 g, 1.2 eq.) is combined with compound 23 (3.1 g, 1.0 eq.) and acetonitrile (ACN, 30 mL). Triethylamine (TEA, 110 mg, 0.05 eq) is added and the reaction is stirred at 25 deg C for 4 hours. Dichloromethane (DCM, 100 mL) is then added and the mixture is washed with aqueous hydrochloric acid (2 x 30 mL) and saturated aqueous sodium bicarbonate (2 x 30 mL). The organic phase is dried over anhydrous sodium sulfate, is filtered and is concentrated to dryness. The residue is purified on silica gel (ethyl acetate/hexane 1/30) giving compound 24 (2.6 g) as a yellow oil. 1H NMR (300 MHz, CDCI3): 6 1.10 (s, 3H), 1.90 (t, 2H), 2.50 (t, 2H), 2.65 (t, 2H), 2.70-2.90 (m, 4H), 3.35 (s, 3H), 3.75 (t, 2H), 4.60 (s, 2H).

### EXAMPLE 12 - Preparation of compound 52 - 5-Methoxymethoxy-pent-1-ene-3-ol (Scheme 10)

Compound 48 (100 g) is reduced to the corresponding alcohol using lithium aluminum hydride as described by Batt, et al. (Eur. J. Org. Chem., 2011, 6039-6055). The resulting diol (13 g, 1 eq.) is added to a mixture of cyclohexane (26 mL), dichloromethane (DCM, 13 mL) and diisopropyl ethylamine (DIEA, 18 g, 1.1 eq.) under nitrogen. Methoxymethyl chloride (1 eq.) is added dropwise and the reaction is stirred at 20 deg C for 12 hours. DCM (100 mL) is then added and the mixture is washed with aqueous hydrochloric acid (2 M, 30 mL) and saturated aqueous sodium bicarbonate (2 x 30 mL). The organic phase is dried over anhydrous sodium sulfate, is filtered and is concentrated to dryness. The residue is purified on silica gel (10% ethyl acetate/hexane) giving the primary MOM ether (compound 52, 4 g) as a yellow oil. 1H NMR (300 MHz, CDCI3): 6 1.75-1.95 (m, 2H), 3.35 (s, 3H), 3.65-3.80 (m, 2H), 4.30-4.35 (m, 1H), 4.65 (s, 2H), 5.10-5.15 (m, 1H), 5.25-5.30 (m, 1H), 5.85-5.95 (m, 1H).

### EXAMPLE 13 - Preparation of compound 23 - 5-Methoxymethoxy-pent-1-ene-3-one (Scheme 10)

Compound 52 (3.5 g, 1.0 eq.) is dissolved in dimethyl sulfoxide (DMSO, 20 mL) under nitrogen. 2-lodoxybenzoic acid (IBX, 9.8 g, 1.5 eq.) is added and the reaction is stirred at 20 deg C for 12 hours. DCM (100 mL) is added and the resulting mixture is washed with saturated aqueous sodium sulfite (30 mL) and saturated aqueous sodium bicarbonate (30 mL). The organic phase is dried over anhydrous sodium sulfate, is filtered and is concentrated to dryness. The residue is purified on silica gel (Ethyl acetate/hexane 1/30) giving the desired compound 23 (3.1 g) as a yellow oil. 1H NMR (300 MHz, CDCI3): 62.90 (t, 2H), 3.35 (s, 3H), 3.90 (t, 2H), 4.65 (s, 2H), 5.90 (d, 1H), 6.20-6.45 (m, 2H).

### EXAMPLE 14 - Preparation of compound 55 (Scheme 10a) - 3-Methoxymethyl propa n-1-01

Cyclohexane (180 mL), dichloromethane (90mL) and diisopropylethylamine (34 g, 1.1 eq.) are combined and propane-1,3-diol (20 g, 1.0 eq.) is added. Methoxymethyl chloride (20.9 g, 0.99 eq.) is added dropwise maintaining the internal reaction temperature at 20 deg C. The reaction is stirred at 20 deg C for 12 hours after which, dichloromethane (100 mL) is added. The mixture is washed with saturated aqueous sodium bicarbonate (2 x 30 mL), is dried over anhydrous sodium sulfate, is filtered and is concentrated to dryness. The residue is purified on silica gel (ethyl acetate/hexane 1/5) giving compound 55 (5 g) as a yellow oil. 1H NMR (300 MHz, CDCI3): 6 1.80-1.90 (m, 2H), 3.40 (s, 3H), 3.70 (t, 2H), 3.80 (t, 2H), 4.65 (s, 2H).

### EXAMPLE 15 - Preparation of compound 56 (Scheme 10a) - 3-Methoxymethyl pro pionaldehyde

Compound 55 (1g, 1.0 eq.) is dissolved in dimethylsulfoxide (10 mL) and 2-lodoxybenzoic acid (IBX, 3.5 g, 1.5 eq.) is added. The reaction is stirred at 20 deg C for 12 hours after which, it is washed with saturated aqueous sodium sulfite (20 mL) and is saturated aqueous sodium bicarbonate (20 mL). The organic phase is dried over anhydrous sodium sulfate, is filtered and is concentrated to dryness. The residue is purified on silica gel (ethyl acetate/hexane 1/20) giving compound 56 (0.3 g, 60% purity) as a yellow oil. 1H NMR (300 MHz, CDCI3): 6 1.80-1.90 (m, 2H), 3.40 (s, 3H), 3.70 (t, 2H), 3.80 (t, 2H), 4.65 (s, 2H).

### EXAMPLE 16 - Preparation of compound 2 (Scheme 11)

Compound 2 is reported by Yamauchi, et al. (Bull. Chem. Soc. Jpn., 2001, 2513-2519). The Scheme 11 sequence for preparation of compound 2 is adapted from Batt, et al. (Eur. J. Org. Chem., 2011, 6039-6055).

### EXAMPLE 17 - Preparation of compound 2 (Scheme 11a)

Propylene glycol (500 g) is combined with benzyl bromide (100 g, 1.0 eq.) under nitrogen. Sodium hydroxide (28 g, 1.2 eq.) is added and the mixture is stirred at 20 deg C for 4 hours. Ethyl acetate (800 mL) is then added and the mixture is washed with water (500 mL). The organic phase is dried over anhydrous sodium sulfate, is filtered and is concentrated to dryness giving the desired crude 3-benzyloxypropanol (100 g) as a yellow oil. 1H NMR (300 MHz, CDCI3): 6 1.85-1.90 (m, 2H), 3.65 (t, 2H), 3.80 (t, 2H), 4.25 (t, 1H), 4.55 (s, 2H), 7.25-7.40 (m, 5H). Crude 3-benzyloxypropanol (100 g, 1.0 eq.) is combined with dimethyl sulfoxide (DMSO, 500 mL) and tetrahydrofuran (THF, 500 mL) under nitrogen. 2-lodoxybenzoic acid (IBX, 253 g, 1.5 eq.) is added and the reaction is stirred at 20 deg C for 12 hours. Ethyl acetate (1500 mL) is then added and the mixture is washed with saturated aqueous sodium sulfite (500 mL) and saturated aqueous sodium bicarbonate (500 mL). The organic phase is washed with anhydrous sodium sulfate, is filtered and is concentrated to dryness. The residue is purified on silica gel (ethyl acetate/hexane 1/20) giving the desired 3-benzyloxypropionaldehyde (30 g) as a yellow oil. 1H NMR (300 MHz, CDCI3): 62.70 (m, 2H), 3.80 (t, 2H), 4.55 (s, 2H), 7.25-7.40 (m, 5H), 9.80 (s, 1H). 3-benzyloxypropionaldehyde (30 g, 1.0 eq.) is dissolved in THF under nitrogen and is cooled to 0 deg C. Vinylmagnesium bromide(1M, 220 mL, 1.2 eq.) is added and the reaction is stirred at 0 deg C for 1 hour. Saturated aqueous ammonium chloride (100 mL) is then added and the mixture is extracted with dichloromethane (DCM, 3 x 100 mL). The organic extracts are dried over anhydrous sodium sulfate, are filtered and are concentrated to dryness giving crude 5-benzyloxy-pent-1-ene-3-ol. 1H NMR (300 MHz, CDCI3): 6 1.75-1.99 (m, 2H), 3.60-3.75 (m, 2H), 4.30-4.40 (m, 1H), 4.50 (s, 2H), 4.70 (s, 1H), 5.10-5.15 (m, 1H), 5.25-5.30 (m, 1H), 5.80-5.95 (m, 1H), 7.25-7.40 (m, 5H). This material is dissolved in DMSO (120 mL) and THF (120 mL) under nitrogen and IBX (65 g, 1.5 eq.) is added. The mixture is stirred at 20 deg C for 12 hours after which, ethyl acetate (500 mL) is added. The resulting mixture is washed with saturated aqueous sodium sulfite (200 mL) and saturated aqueous sodium bicarbonate (200 mL). The organic phase is dried over anhydrous sodium sulfate, is filtered and is concentrated to dryness. The residue is purified on silica gel (ethyl acetate/hexane 1/20) giving the desired 5-benzyloxy-pent-1-ene-3-one (12.7 g) as a yellow oil. 1H NMR (300 MHz, CDCI3): 6 2.95 (t, 2H), 3.80 (t, 2H), 4.55 (s, 3H), 5.85 (d, 1H), 6.20-6.40 (m, 2H), 7.20-7.40 (m, 5H).

### EXAMPLE 18 - Preparation of compound 62 (Scheme 12)

Compound 45 (300 g) is dissolved in dichloromethane (2.4 L) and ethanol (600 mL). The mixture is cooled to -15 deg C and sodium borohydride (20.85 g) is added portionwise while maintaining the reaction temperature at -15 deg C. The reaction is monitored by LCMS until the content of compound 45 was <0.5%. The reaction is quenched with acetic acid (170 mL) and methanol (300 mL) is added. The resulting mixture is concentrated to 25% of its original volume and additional methanol (300 mL) is added. After concentrating to 25% of its original volume, a final portion of methanol (300 mL) is added and the mixture is concentrated to dryness. Dichloromethane (1.5 L) is added and the mixture is stirred for 20 minutes after which, it is filtered and the filter cake is washed with dichloromethane (600 mL). The combined filtrates are concentrated to dryness giving the desired crude alcohol (328 g). This crude material is dissolved in dichloromethane (3.28 L) and is cooled to -50 deg C. Borontrifluoride etherate (83.1 mL) and phosphoric acid (36.9 mL) are added and the mixture is stirred at -50 deg C for 30 minutes. Isobutylene (2.3 kg) is then added at -45 deg C. The mixture is stirred at -40 deg C for 1 hour after which, it is allowed to warm to room temperature. The reaction is monitored by LCMS during this period until the content of the alcohol is <10%. Aqueous ammonium hydroxide (13%, 2.3 L) is then added with vigorous stirring. The layers are separated and the aqueous phase is washed with dichloromethane (1.6 L). The combined organic phases are washed with saturated aqueous ammonium chloride (1.6 L) and brine (1.6 L). The organic phase is dried over anhydrous sodium sulfate, is filtered and is concentrated to dryness. The residue is purified on silica gel giving the desired compound 62 (180 g, 44.3% yield) as a light yellow solid. 1H NMR (300 MHz, CDCI3): 6 5.75 (s, 1H), 3.60 (t, 1H), 2.65-2.75 (m, 1H), 2.45-2.55 (m, 1H), 2.30-2.40 (m, 2H), 1.95-2.05 (m, 2H), 1.65-1.85 (m, 2H), 1.20 (s, 9H), 1.10 (s, 3H).

### EXAMPLE 19 - Preparation of compound 63 (Scheme 12)

Compound 62 (10 g, 1 equivalent) is combined with 1,4-dioxane (50 mL) and water (50 mL). Formaldehyde (37% in water, 3.7 g, 1 equivalent) is added followed by 1,4-diazabicyclo[2.2.2]octane (DABCO, 5.0 g, 1 equivalent). The reaction is stirred at 25-30 deg C for 40 hours and monitored by LCMS until the content of compound 63 was >60%. The reaction is then extracted with isopropanol/dichloromethane (1/3, 2 x 150 mL). The combined organic phases are dried over anhydrous sodium sulfate, are filtered and are concentrated to dryness. The residue is purified on silica gel (25% ethyl acetate/hexane) giving compound 63 (3.15 g, 27.6% yield) as a yellow oil. 1H NMR (300 MHz, DMSO-d6): 6 4.35 (t, 1H), 3.95-4.05 (m, 2H), 3.55-3.65 (m, 1H), 2.60-2.70 (dd, 1H), 2.50-2.60 (m, 1H), 2.45-2.50 (m, 1H), 2.20-2.25 (dd, 1H), 1.85-2.00 (m, 2H), 1.65-175 (m, 2H), 1.15 (s, 9H), 1.00 (s, 3H).

### EXAMPLE 20 - Preparation of compound 63a (Scheme 12a)

Under a nitrogen atmosphere, compound 62 (50 grams) is combined with methyl magnesium carbonate (MMC, 2 M in dichloromethane, 400 mL). The mixture is heated to 115 deg C over 30 minutes with nitrogen bubbling through the reaction. The reaction is stirred for 1 hour at 115 deg C with monitoring by HPLC until the content of compound 63a was > 60%. The reaction is then cooled to 10 deg C and is added dropwise to a mixture of concentrated hydrochloric acid (220 mL) and ice (700 g) with rapid stirring. The layers are separated and the aqueous layer (pH = 3) is washed with methyl tert-butyl ether (MTBE, 500 mL then 250 mL). Water (250 mL) is added to the combined organic layers and the pH is adjusted to 10 on addition of 10% aqueous sodium carbonate solution. The layers are separated and the organic phase is washed with water (250 mL). The pH of the combined aqueous extracts is adjusted to 3 on addition of 10% aqueous hydrochloric acid solution. The resulting mixture is stirred at room temperature for 30 minutes until gas evolution ceases. The resulting solids are collected by filtration and are washed with water (50 mL). The solids are collected and are slurried in petroleum ether (150 mL) for 3 hours. The solids are collected by filtration and are washed with petroleum ether (50 mL). The resulting solids are dried in a vacuum oven at 30 deg C for 5 hours yielding compound 63a (30.2 g, 50.4% yield) as a light yellow solid. 1H NMR (300 MHz, CDCI3): 6 12.6-13.6 (br, 1H), 3.65-3.70 (dd, 1H), 3.10-3.40 (m, 2H), 2.60-2.85 (m, 2H), 2.00-2.15 (m, 2H), 1.75-1.95 (m, 2H), 1.20 (s, 12H).

### EXAMPLE 21 - Preparation of compound 65 (Scheme 12a)

Compound 63a (10 g) is dissolved in anhydrous tetrahydrofuran (100 mL) under a nitrogen atmosphere. Anhydrous 10% palladium on carbon (1 g) is added and the mixture is cooled to 5-10 deg C. The cooled mixture is degassed three times by sequential evacuation and refilling with nitrogen. Following the third evacuation, the reaction vessel is filled with hydrogen. The mixture is stirred under a hydrogen atmosphere at 5-10 deg C for 1 hour and is monitored by LCMS until the reaction is complete. On completion of the hydrogenation, aqueous formaldehyde solution (37%, 20 mL) is added followed by piperidine (0.3 g, 0.10 eq). The mixture is stirred at 5-10 deg C for an additional 1 hour and monitored by LCMS until the reaction is complete. On completion of the reaction, brine (25 mL) and ice (25 g) are added and stirring is continued for 15 minutes. The layers are separated and the organic phase is washed with saturated aqueous sodium bicarbonate (50 mL) and brine (50 mL). The organic phase is dried over anhydrous sodium sulfate, is filtered and is concentrated to dryness. The residue is slurried in methanol (5 mL) at 0 deg C for 10 minutes. On filtration, compound 65 (5 g, 56.3% yield) is isolated as a white solid. 1H NMR (300 MHz, CDCI3): 6 6.95 (s, 1H), 5.00 (s, 1H), 3.55-3.65 (dd, 1H), 2.45-2.60 (m, 2H), 2.35-2.45 (m, 1H), 2.05-2.15 (m, 1H), 1.95-2.05 (m, 1H), 1.55-1.80 (m, 4H), 1.15 (s, 9H), 0.80 (s, 3H).

### EXAMPLE 22 - Preparation of compound 66 (Scheme 12)

Compound 10 (17.7 g, 1.2 equivalents) is dissolved in methanol (75 mL) and sodium methoxide solution (30% in methanol, 1.5 g, 0.2 equivalents) is added. The mixture is cooled to 5-10 deg C under nitrogen. Compound 65 (10 g, 1 equivalent) is dissolved in methanol (25 mL) and the resulting solution is added to the compound 10 solution dropwise over 2 hours while maintaining the reaction temperature between 0-5 deg C. The reaction is stirred at 20-25 deg C overnight after which, sodium hydroxide (5 M in water, 20 mL) is added. The reaction is stirred for an additional 2 hours after which, the methanol is removed under vacuum. Water (100 mL) and toluene (20 mL) are added and the mixture is stirred for 15 minutes. The layers are separated and the pH of the aqueous phase is adjusted to 3 with acetic acid. The aqueous mixture is then washed with ethyl acetate (100 mL and 50 mL). The combined organic extracts are concentrated to dryness and the residue is heated to 80 deg C under vacuum for 3 hours to complete the decarboxylation. The resulting residue is purified on silica gel (5% ethyl acetate/hexane) giving the desired compound 66 (10 g, 60% yield) as a light yellow solid. 1H NMR (300 MHz, DMSO-de): 6 3.8-3.9 (m, 4H), 3.45 (t, 1H), 2.65-2.75 (m, 1H), 2.20-2.50 (m, 6H), 1.85-1.95 (m, 2H), 1.75-1.80 (m, 1H), 1.10-1.65 (m, 8H), 1.25 (s, 3H), 1.10 (s, 9H), 0.80 (s, 3H).

### EXAMPLE 23 - Preparation of compound 67 (ent-19-nortestosterone, Scheme 12)

Compound 66 (10 g, 1 equivalent) is dissolved in ethanol (100 mL) and triethylamine (10 mL) is added. Anydrous 10% palladium on carbon (1.0 g) is added. The mixture is degassed under vacuum and filled with a nitrogen atmosphere. This process of degassing and charging with nitrogen is repeated a total of 3 times. Following the third degassing, the reaction is charged with hydrogen. The reaction is heated to 30 deg C for 6 hours. Hydrochloric acid (6 M in water, 40 mL) is then added and the reaction is heated to reflux for an additional 2 hours. The reaction is cooled to 20-25 deg C and is filtered. The filtrate is collected and the ethanol is removed under vacuum. The resulting aqueous mixture is washed with dichloromethane (3 x 100 mL). The combined washes are concentrated to dryness and the residue is purified on silica gel (25% ethyl acetate/hexane) giving the desired compound 67 (4.70 g, 66.8% yield) as a light yellow solid. 1H NMR (300 MHz, CDCI3): 65.85 (s, 1H), 3.65-3.70 (t, 1H), 2.35-2.50 (m, 2H), 2.20-2.35 (m, 3H), 2.05-2.15 (m, 2H), 1.80-1.90 (m, 3H), 1.40-1.70 (m, 4H), 1.20-1.40 (m, 3H), 0.95-1.20 (m, 3H), 0.80-0.90 (m, 1 H), 0.80 (s, 3H). MS (M+ + 1) 275.1.

### EXAMPLE 24 - Preparation of compound 68 (Scheme 12)

67 (100 g, 1 equivalent) is combined with ethylene glycol (312.2 g, 13.8 equivalents, p-toluenesulfonic acid (1.25 g, 0.02 equivalent) and toluene (3 L) in a 5 L flask that is equipped with a Dean-Stark trap assembly. The mixture is heated to reflux under a nitrogen atmosphere. Reflux is maintained for 3 hours and the reaction is monitored by TLC (50% ethyl acetate/petroleum ether) every hour during this period until all starting material is consuMed. The reaction is then cooled to 20-25 deg C and is poured into saturated aqueous sodium bicarbonate (1.5 L). The layers are separated and the aqueous phase is washed with dichloromethane (2 x 1 L). The combined organic layers are dried over anhydrous sodium sulfate, are filtered and are concentrated to dryness. The crude product is purified on silica gel (petroleum ether/ethyl acetate 100:1 to 20:1 with 0.5% triethylamine) giving compound 68 (96.5 g, 83.2% yield) as a mixture of isomers pertaining to the position of the olefin. 1H NMR (300 MHz, DMSO): 6 5.75 (s, 0.2H), 5.20-5.45 (m, 0.3H), 4.40-4.50 (m, 1H), 3.80-3.90 (m, 4H), 3.40-3.50 (m, 1H), 2.00-2.25 (m, 2H), 1.75-2.00 (m, 5H), 1.45-1.75 (m, 6H), 1.30-1.40 (m, 1H), 1.00-1.30 (m, 6H), 0.65 (s, 3H).

### EXAMPLE 25 - Preparation of compound 14 (Scheme 12)

Compound 68 (96.5 g, 1 equivalent) is combined with acetonitrile (386 mL) under a nitrogen atmosphere. 2-lodoxybenzoic acid (IBX, 170 g, 2 equivalents) is added and the reaction is heated to 50-55 deg C for 3 hours. During this time, the reaction is monitored by TLC (50% ethyl acetate/petroleum ether) every hour until the starting material is consuMed. The reaction is then cooled to 20-25 deg C and the resulting solids are removed by filtration. The filter cake is washed with acetonitrile (2 x 193 mL) and the combined filtrates are concentrated giving crude product. The crude product is purified on silica gel (petroleum ether/ethyl acetate 100:1 to 20:1 with 0.5% triethylamine) giving compound 14 (86.5 g, 87.1% yield) as a mixture of isomers pertaining to the position of the olefin. 1H NMR (300 MHz, DMSO): 65.75 (s, 0.2H), 5.25-5.40 (m, 0.3H), 3.80-3.90 (m, 4H), 2.35-2A5 (m, 1H), 1.80-2.30 (m, 8H), 1.45-1.80 (m, 6H), 1.10-1.45 (m, 5H), 0.80 (s, 3H).

### EXAMPLE 26 - Intraperitoneal administration of PRV-002 attenuates motor and cognitive deficits in a rat model of traumatic brain injury

The goal of this study is to evaluate the motor and cognitive function of rats treated with PRV-002, an analogue of the enantiomer of progesterone, following traumatic brain injury. Male, Sprague-Dawley rats, approximately six weeks of age, received a mid-line cortical impact to induce traumatic brain injury. Rats receive intraperitoneal injections of either vehicle solution (45% cyclodextrin), PRV-002 4mg/kg, or PRV-002 16mg/kg at 15 min., 6h, and 24h post-injury. A sham group, which does not undergo impact or treatment is used as a control. Motor function is evaluated using a neurobehavioral battery, known as neuroscore, at 24h and 48h post-injury. Cognitive function is assessed using the Morris water maze (MWM) - memory score at 48h post-injury. Time spent swimming in close proximity to the wall of the Morris water maze (thigmotaxia) is used to evaluate spatial acquisition deficits and potential TBI-induced anxiety.

Significant motor and cognitive deficits are observed in vehicle-treated rats following injury. Injured rats are treated with either PRV-002 4mg/kg or PRV-002 16mg/kg shows significant improvement in neuroscore - motor performance, at 48h post-injury. Cognitive deficits, is measured by MWM-memory score and time spent in thigmotaxia, are also ameliorated in rats treated with either PRV-002 4mg/kg or PRV-002 16mg/kg. These findings provide support for potential clinical use of PRV-002 for the treatment of concussion and traumatic brain injury.

### Methods

### Animals

Male Sprague-Dawley rats (Charles River, Wilmington, MA), six weeks of age and weighing between 225 - 275 g at the time of injury, are used. Rats are housed in standard Plexiglas cages and are maintained on a 12-12 light cycle with lights on at 0700. Food and water are available ad libitum.

### Injury

Prior to surgery, rats are anesthetized via inhalation with an initial induction of 5% isofluorane. The rat's scalp is shaved and cleaned with a 70% isopropanol solution and 10% betadine solution. During the surgery, anesthesia is maintained at 2.5% isofluorane with oxygen at a rate of 500 - 1000 mL/min. The rat's head is secured in a stereotaxic apparatus and a medial incision is made and the scalp is pulled back with bulldog clips over the frontal bone. A 6 mm circular piece of skull is removed with a Micromotor drill that utilized a removable 6 mm circular drill bit. The bone, above the medial frontal cortex (MFC), is removed using fine, curved tipped forceps, leaving the dura intact. An electrically-controlled injury device with a 3 mm metal impactor is used to produce the traumatic brain injury. A piston is placed on the dura. Electrical signals from the piston to a transducer signal correct placement. The piston is then used to produce a contusion at a depth of 3 mm. This procedure is used extensively by researchers conducting work on traumatic brain injury and represents one of the most consistent and reproducible forms of injury. Following injury the tissue is closed with 4¬0 monofilament sutures. Rats are placed in a heated recovery cage following surgery and are returned to their home cage following recovery.

### Treatment

Rats are randomly placed in one of four treatment groups: 1) sham injury group (SHAM), 2) vehicle-treated injury group (VEHICLE), 3) PRV-002 4mg/kg-treated injury group (PRV-002 4mg/kg), or 4) PRV-002 16mg/kg-treated injury group. Rats receive intraperitoneal injections of either vehicle solution (45% cyclodextrin in sterile water) or PRV-002 solution (PRV-002 powder is dissolved into 45% cyclodextrin solution) at 15 minutes, 6 hours, and 24 hours post-injury.

### Neuroscore

Testing of motor function, using a neurobehavioral battery known as neuroscore is conducted at 24 and 48 hours post-injury. The rats are exposed to a series of four neurobehavioral tests and are observed for abnormal twisting behavior. Rats receive scores from +4 uninjured to (-) nonfunctional for both left and right forelimbs in the forelimb extension task and forelimb paw placement, the left and right hind limbs in hind limb flexion, and left and right sides for the lateral pulsion test. If no twisting is observed the rat would score as normal +1, and if there is twisting present the rat would score as abnormal (-). The total possible score is 33. The testing criteria is as follows:

### Forelimb extension

Suspend the rat by its tail and determine the forelimb extension toward floor.

Score separately for both the left and right forelimb.
∘ +4 Normal: Rat extends both forelimbs fully and equally towards floor
∘ +3 Slightly impaired: There is a slight forelimb flexion
∘ +2 Moderately impaired: There is moderate forelimb flexion
∘ +1 Severely impaired: There is severe forelimb flexion
∘ - Nonfunctional: Forelimb remains tucked close to body.

### Lateral Pulsion

During free walking, gently push the rat to the left and right side and determine the decrease in resistance to lateral pulsion. Score for both the left and right side of the rat.
∘ +4 Normal: Rat should resist equally when pushed to each side.
∘ +3 Slightly impaired: Rat maintains moderate resistance
∘ +2 Moderately impaired: Rat maintains slight resistance
∘ +1 Severely impaired: Rat does not resist when pushed
∘ - Non-functional: Rat does not resist when pushed and falls to its side

### Forelimb Paw Placement

Suspend the rat by its tail and with a slight swinging motion observe the ability of the rat to grasp the object with the right and left paw. Score separately for both the left and right forelimb.
∘ +4 Normal: Rat can strongly grasp the object with both paws
∘ +3 Slightly impaired: Rat weakly grasps the object with paw misplacement
∘ +2 Moderately impaired: Rat is weak and unable to maintain grasp of the object
∘ +1 Severely impaired: Rat is unable to grasp the object
∘ - Nonfunctional: Rat shows no attempt to grasp the object

### Hind limb Flexion

Hold the rat by its tail and lift the hind limbs off of the ground. Determine the hind limb flexion for both the right and left limbs.
∘ +4 Normal: Rats have normal extension of hind limbs, no crossing or splaying
∘ +3 Slightly impaired: hind limbs have slight deviation from normal extension, slight clasping or splaying of hind limbs
∘ +2 Moderately impaired: Moderate crossing over or splaying of hind limbs
∘ +1 Severely impaired: Severe deviation from normal extension with severe crossing over or splaying of hind limbs
∘ - Nonfunctional: Hind limbs are crossed or splayed with no normal extension or function

### Twisting

When the rat is suspended, observe if there is twisting
∘ +1 Normal: no twisting
∘ - Abnormal: twisting

Morris Water Maze - Memory Score

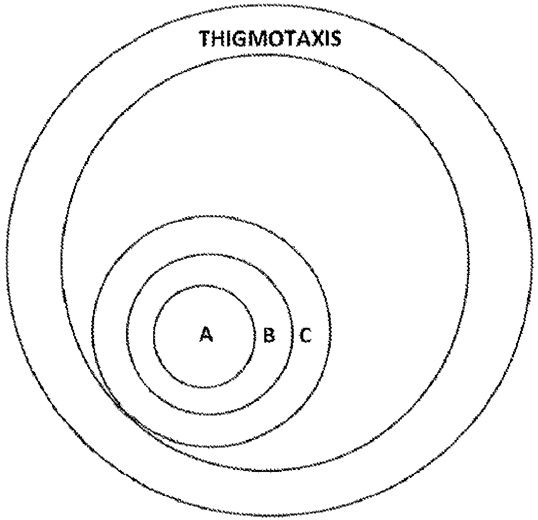

Prior to injury, rats are trained to find a hidden escape platform submerged in location A in a circular pool of water. Forty-eight hours after injury, the platform is removed from the pool and the rats are given two, 60 seconds trials in the pool. Uninjured sham (normal) animals will remember the location of the platform and spend most of their time swimming through and around Zone A. Brain-injured animals whose memory is damaged by the TBI typically swim randomly around the pool, not remembering the location of the hidden platform. The amount of time spent swimming in concentric rings radiating from the escape platform area (zones A, B, and C, respectively) is measured and used to calculate the memory score. The Morris Water Maze memory score is calculated using the equation: (zone A x 20)+(zone B x 5)+(zone C) = memory score , where zones A, B, and C are annuli of increasing size that encompass and surround the area that formerly held the escape platform.

### Morris Water Maze - Thigmotaxia

Thigmotaxis is a measure of the amount of time rats spend "wall hugging" or swimming around the edge of the tank. Time spent traveling in the thigmotaxia area is measured and is indicative of high anxiety and spatial acquisition deficits in injured animals.

### Statistical Analysis

A one-way analysis of variance (ANOVA) is used to evaluate group differences in MWM memory score and MWM thigmotaxia. When warranted, post-hoc analysis of pair-wise comparisons is carried out using Fisher's Protected Least Significant Differences (PLSD) test.

Neuroscore data is analyzed using the Kruskall-Wallis test to evaluate group differences. When warranted, pair-wise comparisons are carried out using the Mann-Whitney U Test.

### Results

### Neuroscore

Kruskal-Wallis tests are carried out to evaluate group differences on median neuroscore at 24h and 48h post-injury. These tests failed to reveal significant differences at 24h [x2 (3, n = 32) = 4.218, p = 0.239] (figure 1) but do reveal significant group differences at 48h post-injury [x2 (3, n=32) = 16.066, p = 0.001] (figure 2). Pair-wise comparisons are carried out using the Mann-Whitney U test at both 24h (table 1) and 48h (table 2) time points. Rats treated with either PRV-002 4mg/kg or PRV-002 16mg/kg have significantly better motor performance, compared to vehicle-treated rats, at 48h post-injury.

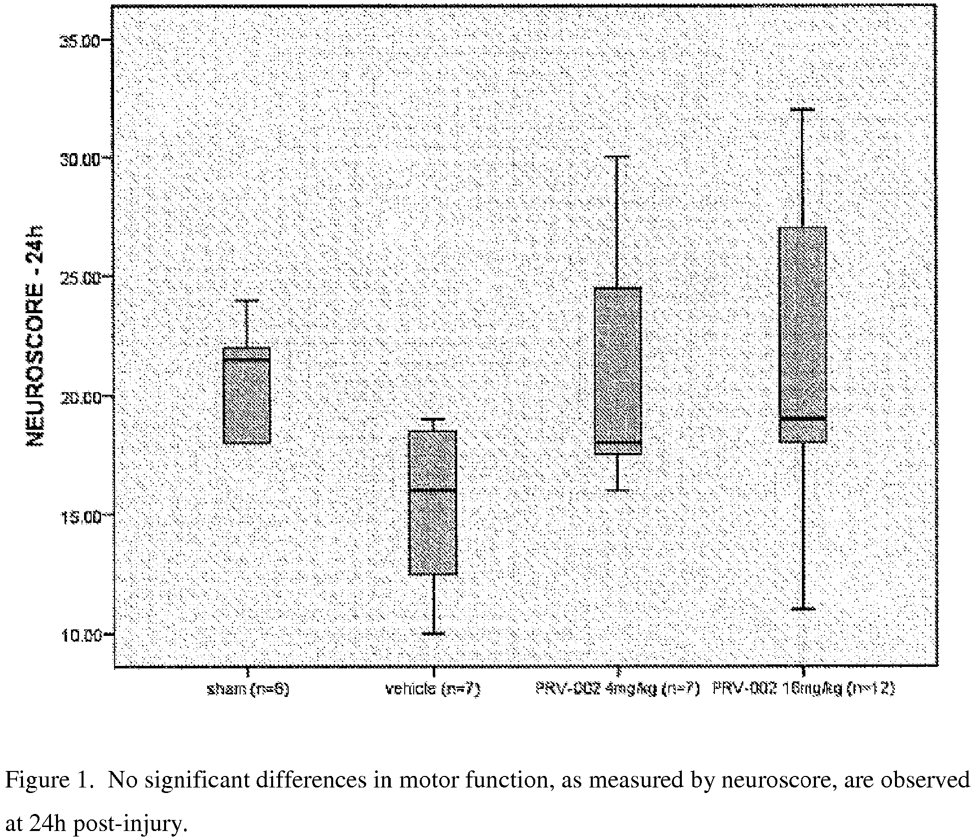

**Table 1. Neuroscore Pair-Wise Comparisons - 24h post-injury**

| | **VEHICLE** | **PRV-002 4mg/kg** | **PRV-002 16mg/kg** |
|---|---|---|---|
| **SHAM** | **U = 1.0, p = 0.084** | **U = 19.0, p = 0.772** | **U = 34.0, p = 0.848** |
| **VEHICLE** | | **U = 14.0, p = 0.177** | **U = 22,0, p = 0.087** |
| **PRV-002 4mg/kg** | | | **U = 33.0, p = 0.439** |

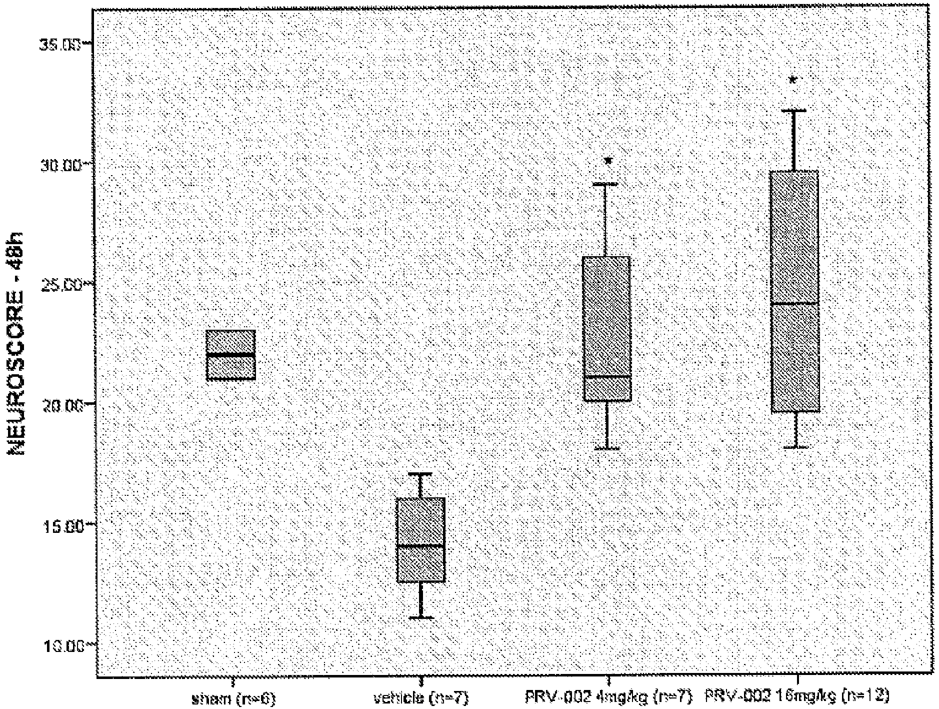

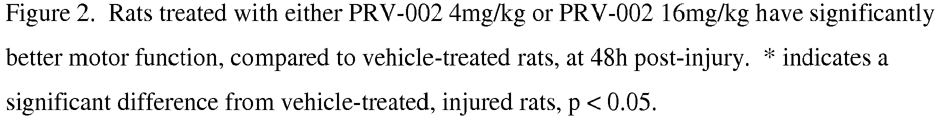

**Table 2. Neuroscore Pair-Wise Comparisons - 48h post-injury**

| | **VEHICLE** | **PRV-002 4mg/kg** | **PRV-002 16mg/kg** |
|---|---|---|---|
| **SHAM** | U = 1.0, p = 0.004* | U = 19.5, p = 0.829 | U = 28.0, p = 0.452 |
| **VEHICLE** | | U = 0.0, p = 0.002* | U = 0.0, p =0.001* |
| **PRV-002 4mg/kg** | | | U = 35.5, p = 0.580 |

| | | | |
|---|---|---|---|
| * Indicates a significant difference, p < 0.05 | | | |

### Morris Water Maze - Memory Score

A one-way analysis of variance (ANOVA) is used to evaluate group differences in MWM memory score. Post-hoc analysis of pair-wise comparisons is carried out using Fisher's Protected Least Significant Differences (PLSD) test. Analysis reveals significant group differences in memory score during both trial 1 [F (3, 32) = 3.863, p 0.019] and trial 2 [F (3, 32) = 3.580, p = 0.026] of the MWM task. Post-hoc analysis shows that vehicle-treated injured rats have significantly worse cognitive function than sham, PRV-002 4mg/kg-, and PRV-002 16mg/kg-treated rats during both trials of the MWM task (figure 3).

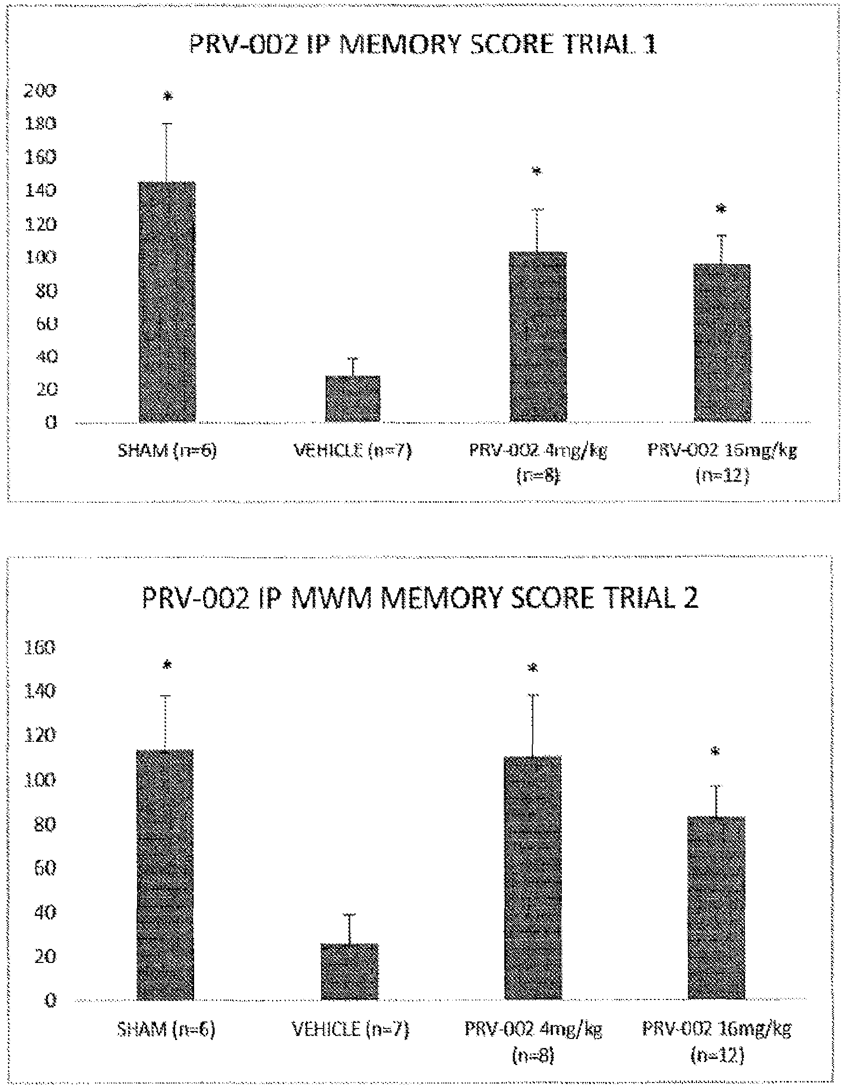

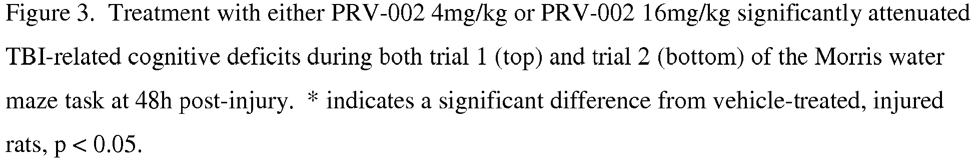

### Morris Water Maze - Time Spent in Thigmotaxia

A one-way analysis of variance (ANOVA) is used to evaluate group differences in time spent in thigmotaxia during the MWM task. Post-hoc analysis of pair-wise comparisons is carried out using Fisher's Protected Least Significant Differences (PLSD) test. Analysis reveals significant group differences in time spent in thigmotaxia during both trial 1 [F (3, 32) = 3.329, p = 0.033] and trial 2 [F (3, 32) = 4.7665, p = 0.008] of the MWM task. Post-hoc analysis shows that vehicle-treated injured rats spend significantly more time in thigmotaxia than sham, PRV-002 4mg/kg-, and PRV-002 16mg/kg-treated rats during both trials of the MWM task (figure 4).

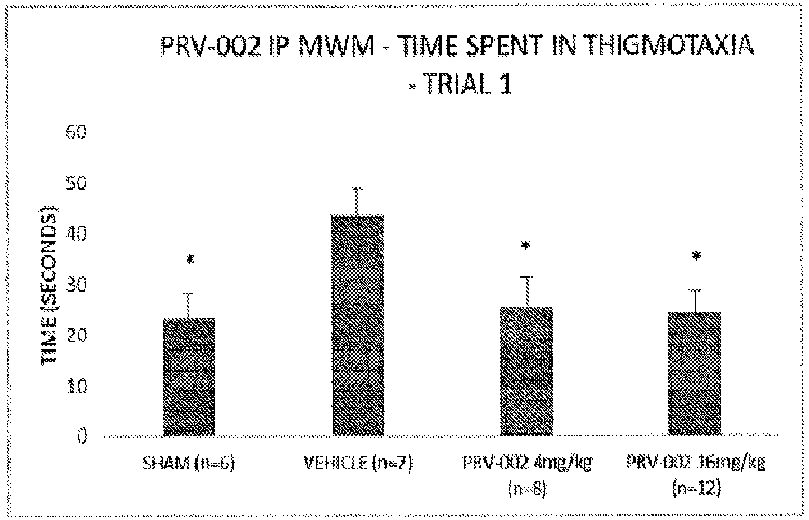

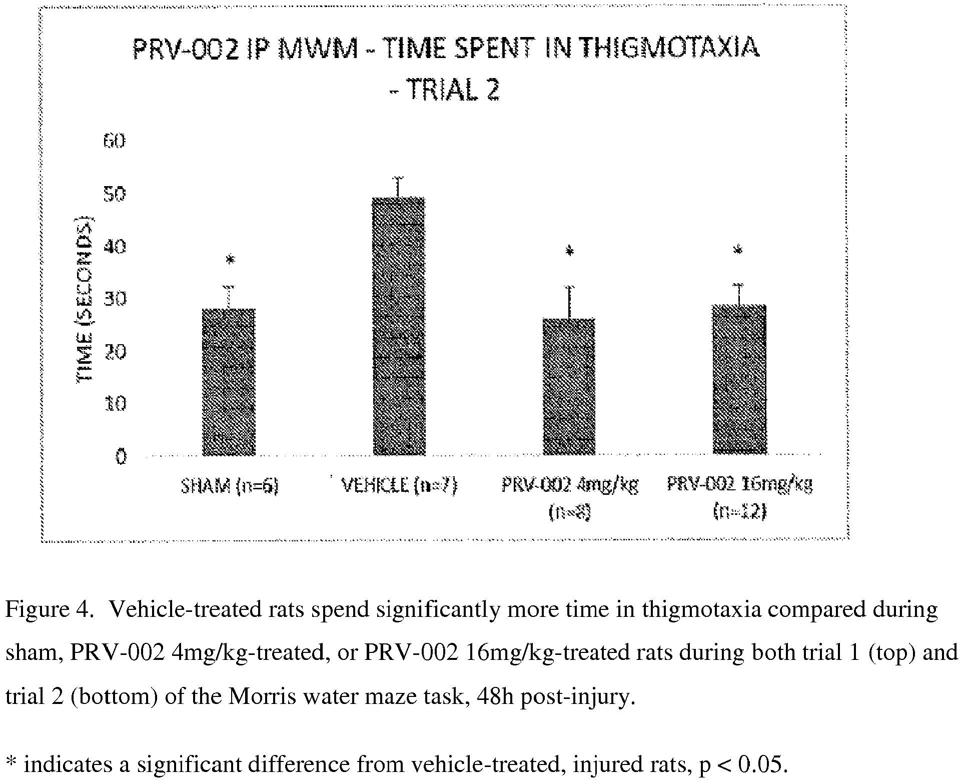

### Discussion

Neuroscore, MWM-memory score, and MWM-time spend in thigmotaxia all reveal significant motor and cognitive deficits in vehicle-treated rats following experimental traumatic brain injury. Though no significant group differences are seen in neuroscore at 24h post-injury, by 48h rats treated with PRV-002 4mg/kg or PRV-002 16mg/kg show significant attenuation of TBI-induced motor function deficits. Rats treated with PRV-002 4mg/kg or PRV-002 16mg/kg show amelioration of TBI-induced cognitive deficits, as measured by the MWM-memory score at 48h hours post-injury. Rats treated with either PRV-002 4mg/kg or PRV-002 16mg/kg spend less time in the thigmotaxia area during the water maze task, compared to vehicle-treated injured rats, indicating a reduction spatial acquisition deficits. The decreased time spent in thigmotaxia may also indicate that treatment with PRV-002 4mg/kg or PRV-002 16mg/kg may induce anxiolytic effects following TBI.

The results of this study reveal the efficacy of PRV-002 in counteracting TBI-induced motor and cognitive deficits in the cortical impact model of TBI in rats. These findings, coupled with previous work investigating the role of PRV-002 in attenuating neurodegeneration and death in cell culture models of TBI, provide support for the use of this compound for the treatment of concussion and TBI in humans. Studies investigating changes in protein expression in the brains of rats that are treated with either vehicle solution or PRV-002 following experimental brain injury will help to elucidate the mechanism by which this compound exerts in neuroprotective effect.

### Example 27 - Intranasal administration of PRV-002 attenuates motor and cognitive deficits in a rat model of traumatic brain injury

The goal of this study is to evaluate the motor and cognitive function of rats treated via intranasal administration with PRV-002, an analogue of the enantiomer of progesterone, following traumatic brain injury. Prior to the initiation of the treatment study, an anatomical evaluation is performed using PRV002 labeled with Evans Blue dye to determine the optimal intranasal/intracerebral penetration of compound using intranasal administration via a miniature atomizer vs. a manual pipette. Post-mortem evaluation determined a clear advantage of the miniature atomizer over the pipette technique with respect to maximal nasal mucosal penetration.

Male, Sprague-Dawley rats, approximately six weeks of age, received a mid-line cortical impact to induce traumatic brain injury. Rats received a intranasal administration, via a miniature atomizer, of either vehicle solution (45% cyclodextrin), PRV-002 0.05 mg/kg (n=4), PRV-002 0.01mg/kg (n=11), PRV002 1mg/kg (n=4) or PRV002 4mg/kg (n=3) at 15 min., 6h, and 24h post-injury. A sham group, which did not undergo impact or receive treatment was used as a control. Motor function is evaluated using a neurobehavioral battery, known as neuroscore, at 24h and 48h post-injury. Cognitive function is assessed using the Morris water maze (MWM) - memory score at 48h post-injury. Time spent swimming in close proximity to the wall of the Morris water maze (thigmotaxia) is used to evaluate spatial acquisition deficits and potential TBI-induced anxiety.

Significant motor and cognitive deficits are observed in vehicle-treated, brain-injured rats following injury. Brain-injured rats treated IN with 4mg/kg PRV002 shows significant improvement in cognitive function (post-traumatic memory) tested at 48 h post-injury/treatment. Time spent in thigmotaxia is also significantly reduced in brain-injured animals receiving IN PRV002 (4mg/kg). Post-traumatic motor deficits at 24 h post-injury are significantly improved in animals treated with either PRV002 (0.1 mg/kg) or PRV002 (4mg/kg). By 48 hr post-injury, brain-injured animals treated with PRV002 (0.05mg/kg), PRV002 (0.1 mg/kg) or PRV002 (4mg/kg) when compared with brain-injured, vehicle-treated animals. These findings provide support for potential clinical use of PRV-002 for the treatment of concussion and traumatic brain injury.

### Methods

### Animals

Male Sprague-Dawley rats (Charles River, Wilmington, MA), six weeks of age and weighing between 225 - 275 g at the time of injury, are used. Rats are housed in standard Plexiglas cages and are maintained on a 12-12 light cycle with lights on at 0700. Food and water are available ad libitum.

### Traumatic Brain Injury Model

Prior to surgery, rats are anesthetized via inhalation with an initial induction of 5% isofluorane. The rat's scalp is shaved and cleaned with a 70% isopropanol solution and 10% betadine solution. During the surgery, anesthesia is maintained at 2.5% isofluorane with oxygen at a rate of 500 -1000 mL/min. The rat's head is secured in a stereotaxic apparatus and a medial incision is made and the scalp is pulled back with bulldog clips over the frontal bone. A 6 mm circular piece of skull is removed with a Micromotor drill that utilized a removable 6 mm circular drill bit. The bone, above the medial frontal cortex (MFC), is removed using fine, curved tipped forceps, leaving the dura intact. An electrically-controlled injury device with a 3 mm metal impactor is used to produce the traumatic brain injury. A piston is placed on the dura. Electrical signals from the piston to a transducer signal correct placement. The piston is then used to produce a contusion at a depth of 3 mm. This procedure is used extensively by researchers conducting work on traumatic brain injury and represents one of the most consistent and reproducible forms of injury. Following injury the tissue is closed with 4¬0 monofilament sutures. Rats are placed in a heated recovery cage following surgery and are returned to their home cage following recovery.

### Treatment

Rats are randomly placed in one of four treatment groups: 1) sham injury group (SHAM-anesthesia and surgical incision without TBI), 2) brain-injured, vehicle-treated injury group (VEHICLE), or TBI followed by intranasal (IN) administration of PRV002 (0.05mg/kg, n=4), PRV002 (0.1mg/kg, n=11), PRV002 (1mg/kg, n=4), or PRV002 (4mg/kg, n=3). Experimental subjects receive an IN spray of either vehicle solution (45% cyclodextrin in sterile water) or PRV-002 solution (PRV-002 powder dissolved into 45% cyclodextrin solution) at 15 minutes, 6 hours, and 24 hours post-injury using a micro atomizer.

### Neuroscore

Testing of motor function, using a neurobehavioral battery known as neuroscore is conducted at 24 and 48 hours post-injury. The rats are exposed to a series of four neurobehavioral tests and are observed for abnormal twisting behavior. Rats receive scores from +4 uninjured to (-) nonfunctional for both left and right forelimbs in the forelimb extension task and forelimb paw placement, the left and right hind limbs in hind limb flexion, and left and right sides for the lateral pulsion test. If no twisting is observed the rat would score as normal +1, and if there is twisting present the rat scores as abnormal (-). The total possible score is 33. The testing criteria is as follows:

### Forelimb extension

Suspend the rat by its tail and determine the forelimb extension toward floor. Score separately for both the left and right forelimb.
∘ +4 Normal: Rat extends both forelimbs fully and equally towards floor
∘ +3 Slightly impaired: There is a slight forelimb flexion
∘ +2 Moderately impaired: There is moderate forelimb flexion
∘ +1 Severely impaired: There is severe forelimb flexion
∘ - Nonfunctional: Forelimb remains tucked close to body.

### Lateral Pulsion

During free walking, gently push the rat to the left and right side and determine the decrease in resistance to lateral pulsion. Score for both the left and right side of the rat.
∘ +4 Normal: Rat should resist equally when pushed to each side.
∘ +3 Slightly impaired: Rat maintains moderate resistance
∘ +2 Moderately impaired: Rat maintains slight resistance
∘ +1 Severely impaired: Rat does not resist when pushed
∘ - Non-functional: Rat does not resist when pushed and falls to its side

### Forelimb Paw Placement

Suspend the rat by its tail and with a slight swinging motion observe the ability of the rat to grasp the object with the right and left paw. Score separately for both the left and right forelimb.
∘ 0 +4 Normal: Rat can strongly grasp the object with both paws
∘ 0 +3 Slightly impaired: Rat weakly grasps the object with paw misplacement
∘ 0 +2 Moderately impaired: Rat is weak and unable to maintain' grasp of the object
∘ o+1 Severely impaired: Rat is unable to grasp the object
∘ o- Nonfunctional: Rat shows no attempt to grasp the object

### Hind limb Flexion

Hold the rat by its tail and lift the hind limbs off of the ground. Determine the hind limb flexion for both the right and left limbs.
∘ 0 +4 Normal: Rats have normal extension of hind limbs, no crossing or splaying
∘ o+3 Slightly impaired: hind limbs have slight deviation from normal extension, slight clasping or splaying of hind limbs
∘ o+2 Moderately impaired: Moderate crossing over or splaying of hind limbs
∘ 0 +1 Severely impaired: Severe deviation from normal extension with severe crossing over or splaying of hind limbs
∘ - Nonfunctional: Hind limbs are crossed or splayed with no normal extension or function

### Twisting

When the rat is suspended, observe if there is twisting
∘ +1 Normal: no twisting
∘ - Abnormal: twisting

### Cognition: Morris Water Maze - Memory Score

### THIGMOTAXIS

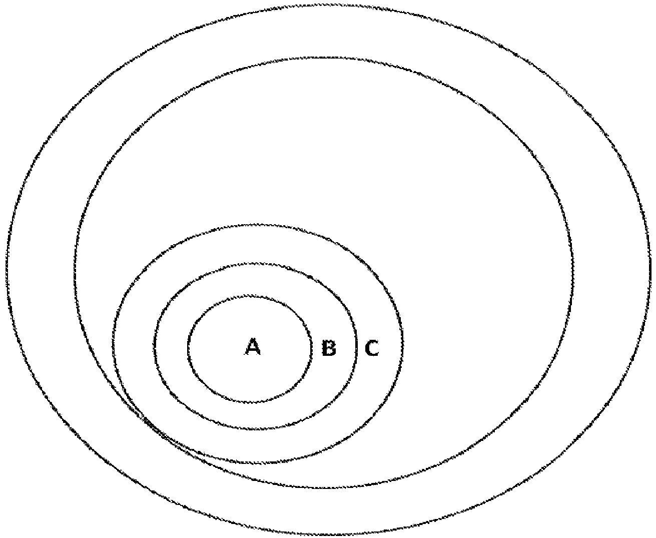

Prior to injury, rats are trained to find a hidden escape platform submerged in location A in a circular pool of water. Forty-eight hours after injury, the platform is removed from the pool and the rats are given two, 60 seconds trials in the pool. Uninjured sham (normal) animals will remember the location of the platform and spend most of their time swimming through and around Zone A. Brain-injured animals whose memory is damaged by the TBI typically swim randomly around the pool, not remembering the location of the hidden platform. The amount of time spent swimming in concentric rings radiating from the escape platform area (zones A, B, and C, respectively) is measured and used to calculate the memory score. The Morris Water Maze memory score is calculated using the equation:
(Zone A x 20) (Zone B x + Zane C = -memory score, where zones A, B, and C are annuli of increasing size that encompass and surround the area that formerly holds the escape platform.

### Morris Water Maze - Thigmotaxia

Thigmotaxis is a measure of the amount of time rats spend "wall hugging" or swimming around the edge of the tank. Time spent traveling in the thigmotaxia area is measured and is indicative of high anxiety and spatial acquisition deficits in injured animals.

### Statistical Analysis

A one-way analysis of variance (ANOVA) is used to evaluate group differences in MWM memory score and MWM thigmotaxia. When warranted, post-hoc analysis of pair-wise comparisons is carried out using Fisher's Protected Least Significant Differences (PLSD) test. Neuroscore data is analyzed using the Kruskall-Wallis test to evaluate group differences. When warranted, pair-wise comparisons are carried out using the Mann-Whitney U Test.

### Results

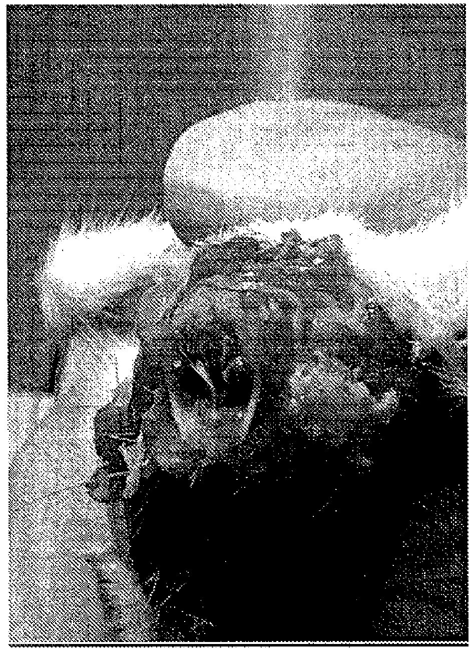

No Evans Blue Dye observable in nasal mucosa using Pipette for IN administration

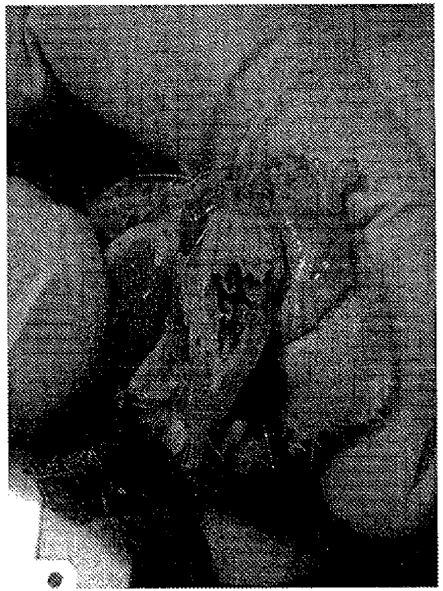

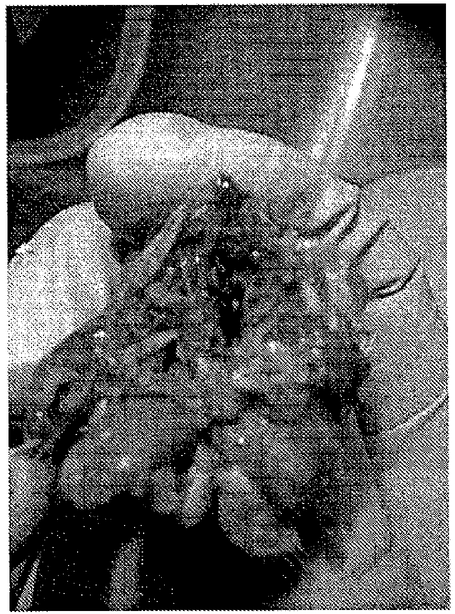

Excellent intranasal penetration observed using micro Atomizer.

### COGNITION

### Morris Water Maze - Memory Score

A one-way analysis of variance (ANOVA) is used to evaluate group differences in MWM memory score. Post-hoc analysis of pair-wise comparisons is carried out using Fisher's Protected Least Significant Differences (PLSD) test. Analysis reveals significant group differences in memory score during trial 1 [F (5, 31) = 4.433, p = 0.005] (figure 1, top) but not during trial 2 [F (5, 31) = 0.928, p = 0.479] (figure 1, bottom) of the MWM task. Post-hoc analysis shows all groups have significantly lower memory scores than PRV-002 4mg/kg-treated rats during trial 1.

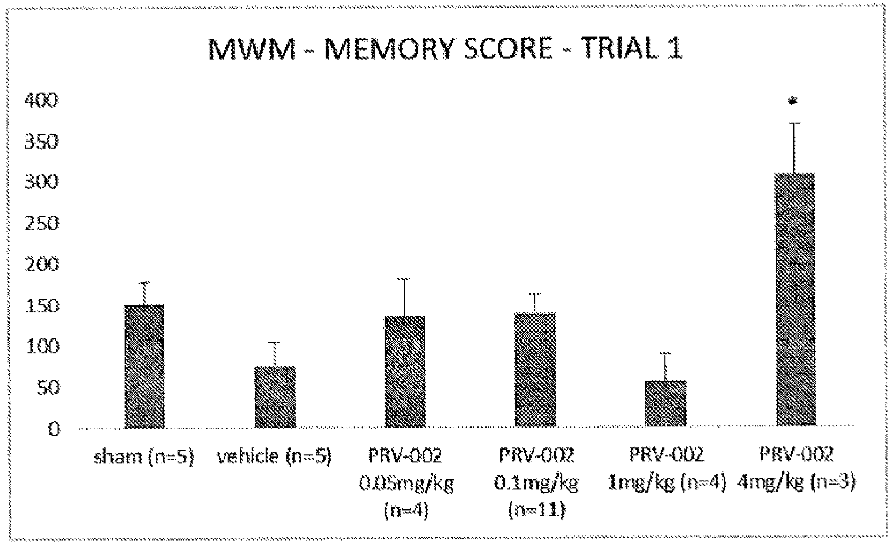

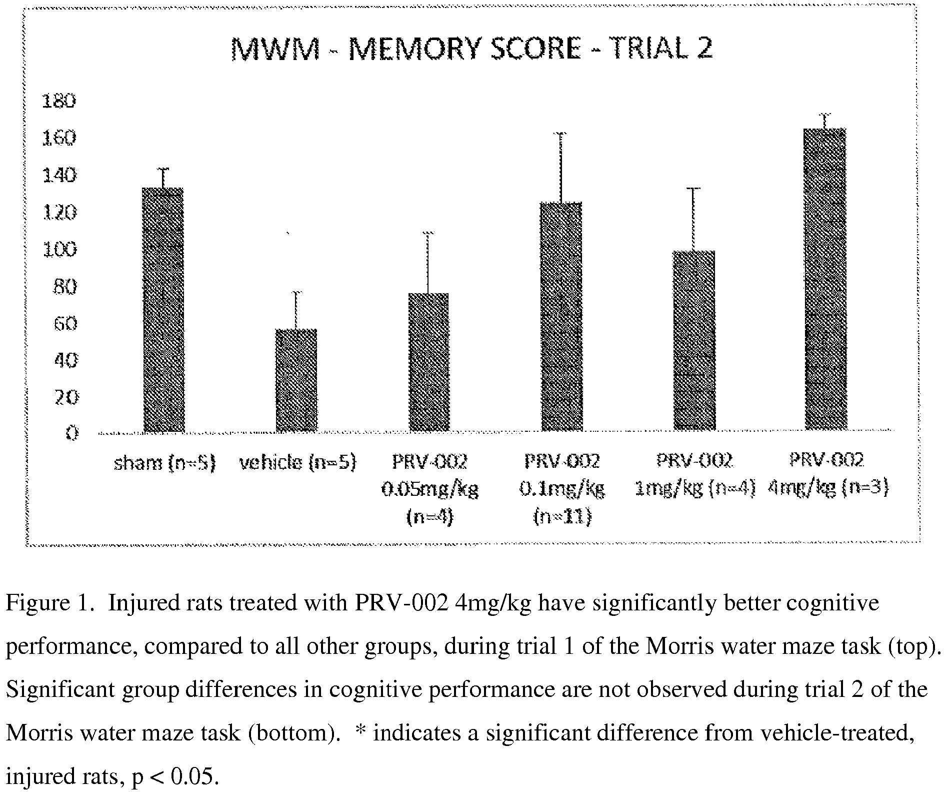

### Morris Water Maze - Time Spent in Thigmotaxia

A one-way analysis of variance (ANOVA) is used to evaluate group differences in time spent in thigmotaxia during the MWM task. Post-hoc analysis of pair-wise comparisons is carried out using Fisher's Protected Least Significant Differences (PLSD) test. Analysis reveals significant group differences in time spent in thigmotaxia during both trial 1 [F (5, 31) = 1.857, p = 0.137] (figure 2, top) and trial 2 [F (5, 31) = 3.103, p = 0.025] (figure 2, bottom) of the MWM task. Post-hoc analysis shows that sham and PRV-002 4mg/kg-treated rats spent significantly less time in thigmotaxia, compared to vehicle-treated rats. 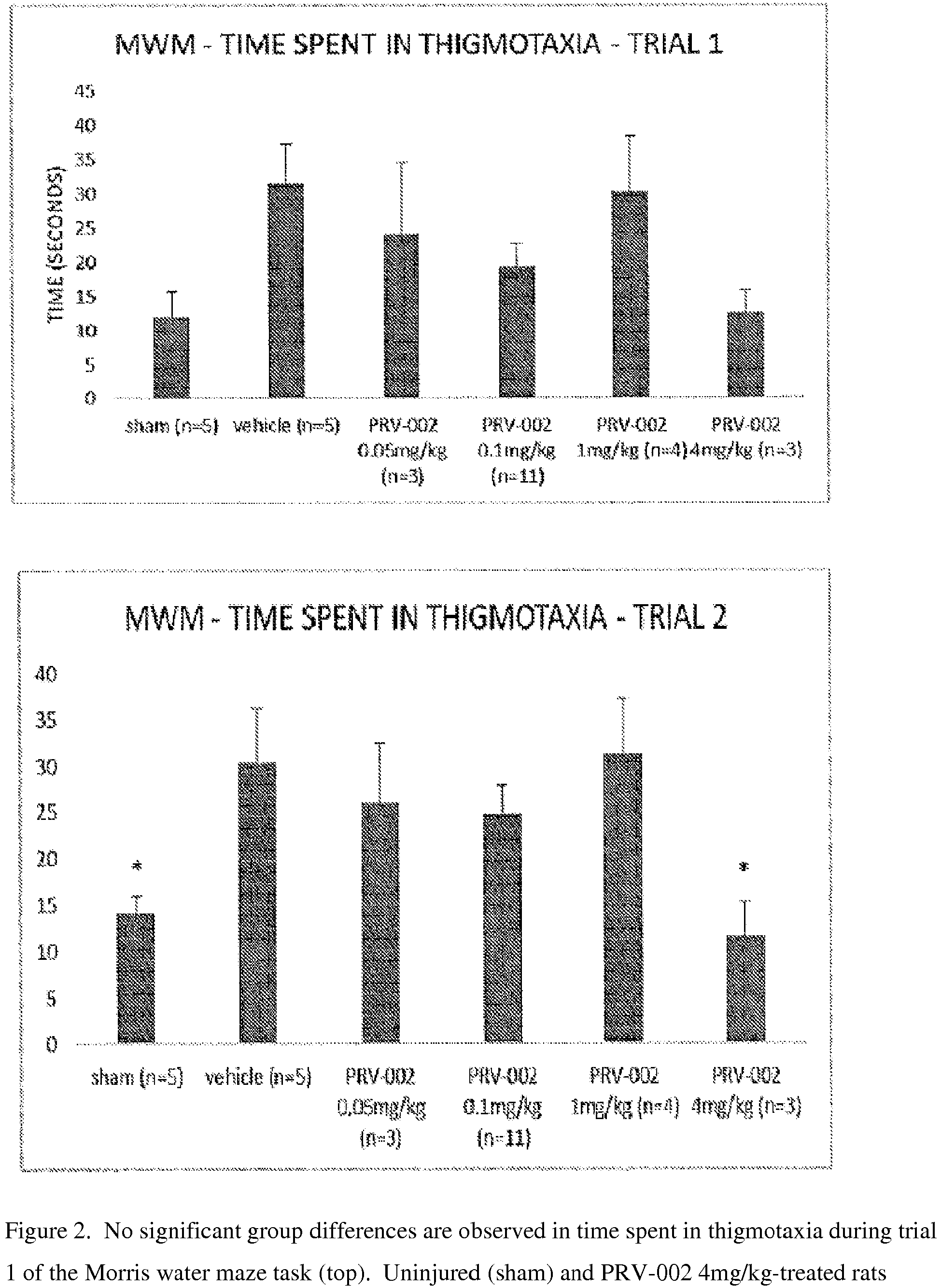 spends significantly less time in thigmotaxia as compared to vehicle-treated injured rats during trial 2 of the Morris water maze task (bottom). Additionally, rats treated with PRV-002 005mg/kg spent significantly more time in thigmotaxia, compared to sham rats, during trial 2. * indicates a significant difference from vehicle-treated, injured rats, p < 0.05.

### MOTOR FUNCTION

### Neuroscore

Kruskal-Wallis tests are carried out to evaluate group differences on median neuroscore at 24h and 48h post-injury. These tests reveal significant differences at 24h [X2 (3, n = 32) = 13.529, p = 0.019] (figure 3) and at 48h post-injury [x2 (3, n=32) = 18.153, p = 0.003] (figure 4). Pair-wise comparisons are carried out using the Mann-Whitney U test at both 24h (table 1) and 48h (table 2) time points. Rats treated with PRV-002 0.1mg/kg or PRV-002 4mg/kg have significantly improved motor function, compared to vehicle-treated rats at 24h post-injury. All PRV-002 treatment groups had motor performance scores that are not significantly different from sham rats at 24h post-injury (table 1). Sham rats and rats treated with either PRV-002 0.05mg/kg, PRV-002 0.1 mg/kg, or PRV-002 4mg/kg have significantly better motor function, as compared to vehicle-treated rats at 48h post-injury. PRV-002 0.05mg/kg- and PRV-002 1 mg/kg- treated rats have significantly worse performance, compared to sham rats at 48h post-injury (table 2).

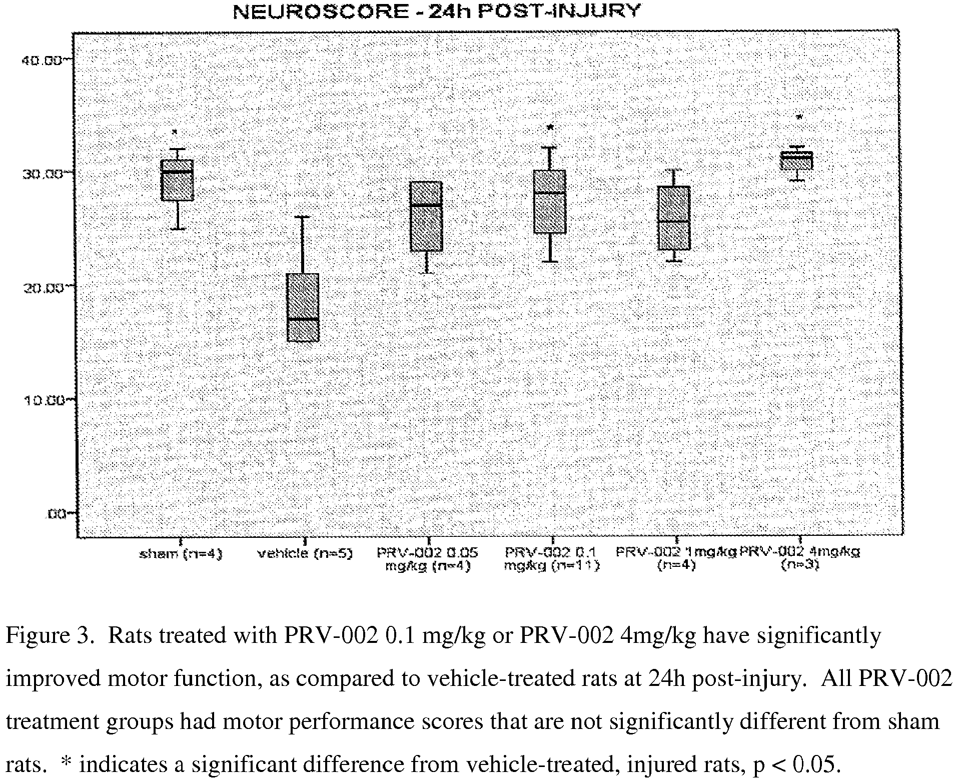

**Table 1. Neuroscore - 24h Post-Injury**

| | **VEHICLE** | **PRV-002 0.05mg/kg** | **PRV-002 0.1 mg/kg** | **PRV-002 1 mg/kg** | **PRV-002 4mg/kg** |
|---|---|---|---|---|---|
| **SHAM** | U = 1.0, p = 0.027* | U = 2.5, p = 0.106 | U = 15.0, p = 0.356 | U = 3.0, p = 0.139 | U = 4.5, p = 0.629 |
| **VEHICLE** | | U = 2.5, p =0.064 | U = 3.5 p = 0.006* | U = 2.0, p = 0.050 | U = 0.0, p = 0.025* |
| **PRV-002 0.05mg/kg** | | | U = 18.0, p = 0.598 | U = 8.0, p = 1.0 | U = 1.0, p = 0.067 |
| **PRV-002 0.1mg/kg** | | | | U = 16.0, p = 0.430 | U = 7.0, p = 0.134 |
| **PRV-002 1mg/kg** | | | | | U = 1.0, p = 0.077 |

| | | | | | |
|---|---|---|---|---|---|
| * indicates a significant difference, p < 0.05 | | | | | |

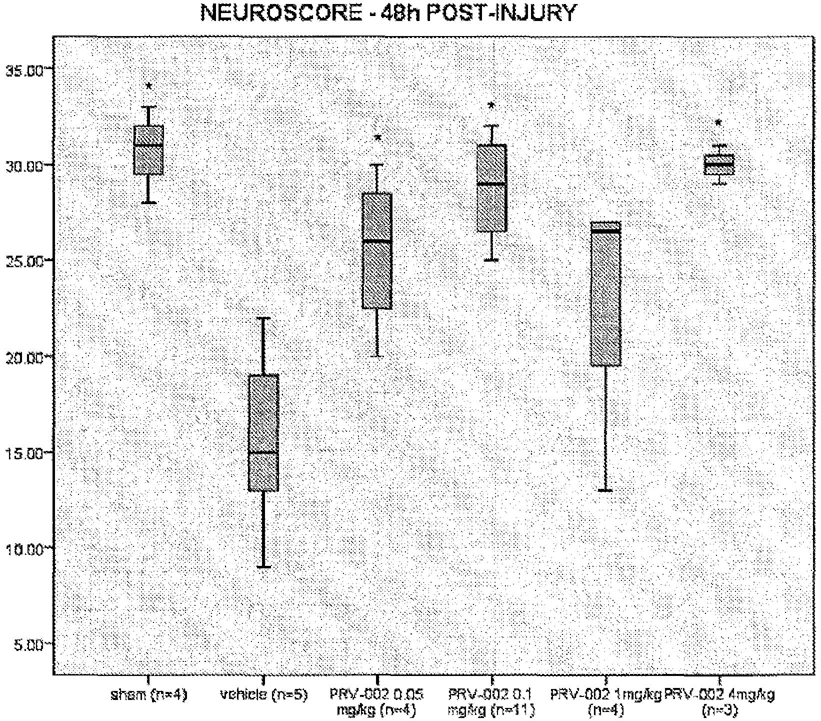

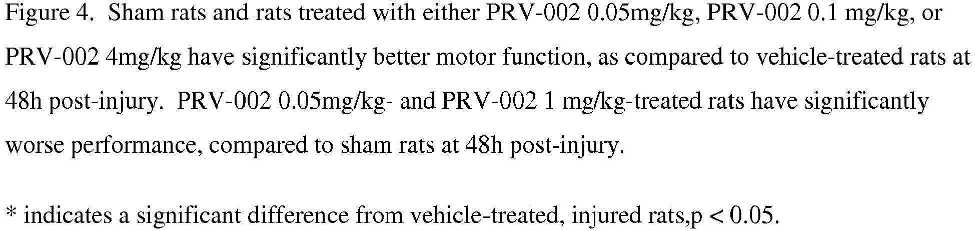

**Table 2. Neuroscore - 48h Post-Injury**

| | **VEHICLE** | **PRV-002 0.05mg/kg** | **PRV-002 0.1mg/kg** | **PRV-002 1mg/kg** | **PRV-002 4mg/kg** |
|---|---|---|---|---|---|
| **SHAM** | U = 0.0, p = 0.014* | U = 1.0, p = 0.042* | U = 12.5, p = 0.209 | U = 0.0, p = 0.019* | U = 4.0, p = 0.463 |
| **VEHICLE** | | U = 1.0, p =0.027* | U = 0.0 p = 0.002* | U = 3.5, p = 0.108 | U = 0.0, p = 0.025* |
| **PRV-002 0.05mg/kg** | | | U = 11.0, p = 0.149 | U = 7.0, p = 0.767 | U = 1.5, p = 0.108 |
| **PRV-002 0.1mg/kg** | | | | U = 9.0, p = 0.086 | U = 12.0, p = 0.479 |
| **PRV-002 1mg/kg** | | | | | U = 0.0, p = 0.032* |

| | | | | | |
|---|---|---|---|---|---|
| * indicates a significant difference, p < 0.05 | | | | | |

### Discussion

Neuroscore, MWM-memory score, and MWM-time spent in thigmotaxia all reveal significant motor and cognitive deficits in brain-injured, vehicle-treated rats following experimental traumatic brain injury. Brain-injured rats treated IN with 4mg/kg PRV002 show significant improvement in cognitive function (post-traumatic memory) tested at 48 h post-injury/treatment. Time spent in thigmotaxia is also significantly reduced in brain-injured animals receiving IN PRV002 (4mg/kg). The decrease in time spent in thigmotaxia may also indicate that IN treatment with PRV-002 may induce anxiolytic effects following TBI.

Post-traumatic motor deficits at 24 h post-injury are significantly improved in animals treated with either PRV002 (0.1mg/kg) or PRV002 (4mg/kg). By 48 hr post-injury, brain-injured animals treated with PRV002 (0.05mg/kg), PRV002 (0.1mg/kg) or PRV002 (4mg/kg) show significantly improved motor function when compared with brain-injured, vehicle-treated animals. The results of this study reveal the efficacy of PRV-002 in counteracting TBI-induced motor and cognitive deficits in the cortical impact model of TBI in rats.

These observations, coupled with previous work showing improvement of cognitive and motor function following systemic (intraperitoneal) administration of PRV002 and studies investigating the role of PRV-002 in attenuating neurodegeneration and death in cell culture models of TBI, provide support for the use of this compound for the treatment of concussion and TBI in humans. Studies investigating changes in protein expression in the brains of rats treated with either vehicle solution or PRV-002 following experimental brain injury, coupled with MRI studies, will help to elucidate the mechanism(s) by which this compound exerts in neuroprotective effect in the injured brain following TBI.

### Example 28

An Example of an ent-19-norprogesterone (PRV-002) solution that is used in accordance with Examples 26 and 27.

| Reagent | Amount added | Mix time, min | Resultant solution | Total volume after solubilization | Final Calculated concentration |
|---|---|---|---|---|---|
| Solution 1 | | | | | |
| 2-Hydroxypropyl-β -cyclodextrin (H107-Sigma-Aldrich) | 2.5 g | | | ∼ 7 ml | 35.8% |
| Water | 5 ml | 10 | Clear | | |

| PRV-002 Formulation | | | | | |
|---|---|---|---|---|---|
| Solution 1 | 1 ml | | | ∼ 1 ml | 35.8% |
| + PRV-002 ent-19-norpropesterone | 30 mg | 60 | Hazy | | |
| + Solution 1 | 0.1 ml | 120 | Hazy | ∼ 1.1 ml | |
| + Solution 1 | 0.1 ml | O/N | Hazy to Clear | ∼ 1.2 ml | |
| + Solution 1 | 0.1 ml | 120 | Mostly clear with slight haziness* | ∼ 1.3 ml | 23 mg/ml |
| | | | | | |
| | | | | | |

| | | | | | |
|---|---|---|---|---|---|
| *- No precipitation is observed after O/N at RT. Apparent solubility of PRV-002 in 35.8% of 2-hydroxypropyl-β-cyclodextrin is about 23 mg/ml. | | | | | |

The PRV-002 solution is prepared by adding about 30 mg of PRV-001 compound to about 1 ml of 35.8% 2-hydroxypropyl-β-cyclodextrin Solution is hazy after mixing for about 60 min. Then about 0.1 ml of about 35.8% 2-hydroxypropyl-β-cyclodextrin is added to about 1 ml of PRV-002-Cyclodextrin mixture. Solution is hazy after mixing for about 120 min. Additional 0.1 ml of about 35.8% 2-hydroxypropyl-β-cyclodextrin is added to about 1.1 ml of PRV-002-Cyclodextrin mixture and left on mixing overnight (0/N). Next day resultant solution is notably clearer but still hazy. About 0.1 ml more of about 35.8% 2 -hydroxypropyl-β-cyclodextrin is added to about 1.2 ml of PRV-002-Cyclodextrin mixture. Addition of another about 0.3 ml (0.1+0.1+0.1) aliquot of about 35.8% 2-hydroxypropyl-β-cyclodextrin only slightly improves the clarity of PRV-002 solution. It is believed that PRV-002 is in solution at about 23mg/mi, and slight haziness is some sort of an artifact.

### Reference List

1. US Publication No. US 2005/0187188 to Stein et al., published August 25, 2005 and entitled "Methods for the Treatment of a Traumatic Central Nervous System Injury"
2. US Publication No. US 2007/0078117 to Hoffman et al., published April 5, 2007 and entitled "Methods for the Treatment of a Traumatic Central Nervous System Injury"
3. US Publication No. US 2008/0318914 to Hoffman et al., published December 25, 2008 and entitled "Methods for the Treatment of a Traumatic Central Nervous System Injury"
4. US Publication No. US 2009/0221544 to Stein et al., published September 3, 2009 and entitled "Methods for the Treatment of a Traumatic Central Nervous System Injury via a Tapered Administration Protocol"
5. US Publication No. US 2009/0325920 to Hoffman et al., published December 31, 2009 and entitled "Methods for the Treatment of a Traumatic Central Nervous System Injury"
6. US Publication No. US 2011/0306579 to Stein et al., published December 15, 2011 and entitled "Methods of Neuroprotection using Neuroprotective Steroids and a Vitamin D"
7. R. J. Auchus et al. "The Enantiomer of Progesterone (ent-progesterone) is a Competitive Inhibitor of Human Cytochromees P450c17 and P450c21," Archives of Biochemistry and Biophysics, 409:134-144 (2003)
8. S. D. Rychnovsky et al. "Synthesis of ent-Cholesterol, the Unnatural Enantiomer," J. Org. Chem. 57:2732-2736 (1992)
9. S. Talengaonkar et al. "Intranasal Delivery: An Approach to Bypass the Blood Brain Barrier," Indian J. Pharmacol, 36(3):140-147 (2004)
10. H. Nemoto et al. " First Enantioselective Total Synthesis of (+)-Cortisone," J. Org. Chem. 55: 5625-5631 (1990)
11. W. S. Johnson et al. "Synthesis of dl-Progesterone," Journal of the American Chemical Society, V93(17):4332-4334 (1971)
12. M. Weimar et al. "Enantioselective Synthesis of (+)-Estrone Exploiting a Hydrogen Bond-Promoted Diels-Alder Reaction," J. Org. Chem, 75:2718-2721 (2010)
13. Herrmann et al. "Formal Total Synthesis of (±)-Estrone and Zirconocene-Promoted Cyclization of 2-Fluoro-1-7-octadienes and Ru-Catalyzed Ring Closing Metathesis," J. Org. Chem, 73:6202-6206 (2008)
14. Q. Hu et al. "Simple, Catalytic Enantioselective Synthesis of Estrone and Desogestrel," J. Am Chem Soc, 126:5984-5986 (2004)
15. Y. Horiguchi et al. "Total Synthesis of (±)-Cortisone. Double Hydroxylation Reaction for the Construction of Corticoid Side Chain," J. Org. Chem., 51: 4323-4325 (1986)
16. US Patent Application No. 13/645,854, invented by VanLandingham et al. and entitled "Prophylactic and Post-Acute Use of Progesterone to Better Outcomes Associates with Concussion"
17. US Patent Application No. 13/645,881, invented by VanLandingham et al. and entitled "Nasal Delivery Mechanism for Prophylactic and Post-Acute Use for Progesterone and/or Its Enantiomer for Use in Treatment of Mild Traumatic Brain Injuries"
18. US Patent Application No. 13/645,925, invented by VanLandingham et al. and entitled "Prophylactic and Post-Acute Use of Progesterone in Conjunction with its Enantiomer for Use in Treatment of Mild Traumatic Brain Injuries"
19. International PCT Patent Application No. PCT/US2012/59030, invented by VanLandingham et al. and entitled "Prophylactic and Post-Acute Use of Progesterone to Better Outcomes Associated with Concussion"
20. International PCT Patent Application No. PCT/US2012/59087, invented by VanLandingham et al. and entitled "Nasal Delivery Mechanism for Prophylactic and Post-Acute Use for Progesterone and/or Its Enantiomer for Use in Treatment of Mild Traumatic Brain Injuries"
21. International PCT Patent Application No. PCT/US2012/59083, invented by VanLandingham et al. and entitled "Prophylactic and Post-Acute Use of Progesterone in Conjunction with its Enantiomer for Use in Treatment of Mild Traumatic Brain Injuries"
22. Bosch, M.P.; Camps, F.; Coll, J.; Guerrero, T.; Tatsuoka, T.; Meinwald, J. J. Org. Chem., 51:773 (1986)
23. Tsunoda, T.; Suzuki, M.; Noyori, R. Tetrahedron Lett., 21:1357 (1980)
24. Yamauchi, Noriaki; Natsubori, Yoshiaki; Murae, Tatsushi Bulletin of the Chemical Society of Japan, 73(11):2513-2519 (2000)
25. Das, Biswanath; Banerjee, Joydeep; Chowdhury, Nikhil; Majhi, Anjoy; Holla, Harish, Synlett, 12:1879-1882 (2006).
26. Liu, Hsing-Jang; Ly, Tai Wei; Tai, Chia-Liang; Wu, Jen-Dar; Liang, Jinn-Kwei; Guo, Jiunn-Cheh; Tseng, Nai-Wen; Shia, Kak-Shan. Tetrahedron, 59(8):1209-1226 (2003).
27. Tai, Chia-Liang; Ly, Tai Wei; Wu, Jen-Dar; Shia, Kak-Shan; Liu, Hsing-Jang. Synlett, (2), 214-217 (2001).
28. Jiro Tsuji, Hideo Nagashima, and Hisao Nemoto, "General Synthetic Method for the preparation of Methyl Ketones from Terminal Olefins: 2-Decanone", Org. Synth.; Coll. Vol. 7: 137 (1990).
29. Basavaiah, D.; Rao, A. J.; Satyanarayana, T. Chem. Rev., 103:811 (2003)
30. Batt, Frederic and Fache, Fabienne, European Journal of Organic Chemistry, (30), 6039-6055, S6039/1-S6039/46 (2011)
31. Song, J. and Hollingsworth, R. I., Tetrahedron: Asymmetry, 12(3):387-391 (2001)
32. Djebaili, M., et al.: The neurosteroids progesterone and allopregnanolone reduce cell death, gliosis, and functional deficits after traumatic brain injury in rats. J Neurotrauma, 22:106-118 (2005)
33. Stein, D.G., Brain damage, sex hormones and recovery: a new role for progesterone and estrogen? Trends Neurosci, 24(7):386-91 (2001).
34. Stein, D.G., Sex differences in brain damage and recovery of function: experimental and clinical findings. Prog Brain Res, 161:339-51 (2007).
35. Pettus, E.H., D.W. Wright, D.G. Stein, and S.W. Hoffman, Progesterone treatment inhibits the inflammatory agents that accompany traumatic brain injury. Brain Res, 1049(1):112-9 (2005).
36. Shear, D.A., R. Galani, S.W. Hoffman, and D.G. Stein, Progesterone protects against necrotic damage and behavioral abnormalities caused by traumatic brain injury. Exp Neurol, 178(1):59-67 (2002).
37. Guo, Q., Baronne, L., Hoffman, S.W., Guennoun, R., Stein, D.G., Progesterone's Effects on Aquaporin-4 Expression after Traumatic Brain Injury In Male Rats. J Neurotrauma, 21:1303 (2004).
38. McCullough, L.D. and P.D. Hum, Estrogen and ischemic neuroprotection: an integrated view. Trends Endocrinol Metab, 14(5):228-35 (2003).
39. Stein, D.G. and S.W. Hoffman, Concepts of CNS plasticity in the context of brain damage and repair. J Head Trauma Rehabil, 18(4):317-41 (2003).
40. Stein, D.G. and S.W. Hoffman, Estrogen and progesterone as neuroprotective agents in the treatment of acute brain injuries. Pediatr Rehabil, 6(1):13-22 (2003).
41. Schumacher, M., S. Weill-Engerer, P. Liere, F. Robert, R.J. Franklin, L.M. Garcia-Segura, J.J. Lambert, W. Mayo, R.C. Melcangi, A. Parducz, U. Suter, C. Carelli, E.E. Baulieu, and Y. Akwa, Steroid hormones and neurosteroids in normal and pathological aging of the nervous system. Prog Neurobiol, 71(1):3-29 (2003).
42. Roof, RI., R. Duvdevani, J.W. Heyburn, and D.G. Stein, Progesterone rapidly decreases brain edema: treatment delayed up to 24 hours is still effective. Exp Neurol, 138(2):246-51 (1996).
43. Roof, R.L., Stein, D. G., Progesterone treatment attenuates brain edema following contusion injury in male and female rats. Restor. Neurol. Neurosc, 4:425-427 (1992).
44. Roof, RI., Fritts, M.E., Progesterone metabolites may mediate its neuroprotective effects after traumatic brain injury. Neurotrauma, 14:760 (1997).
45. Djebaili, M., S.W. Hoffman, and D.G. Stein, Allopregnanolone and progesterone decrease cell death and cognitive deficits after a contusion of the rat pre-frontal cortex. Neuroscience, 123(2):349-59 (2004).
46. VanLandingham, J.W., M. Cekic, S. Cutler, S.W. Hoffman, and D.G. Stein, Neurosteroids reduce inflammation after TBI through CD55 induction. Neurosci Lett, 425(2):94-8 (2007).
47. Guo, Q., I. Sayeed, L.M. Baronne, S.W. Hoffman, R. Guennoun, and D.G. Stein, Progesterone administration modulates AQP4 expression and edema after traumatic brain injury in male rats. Exp Neurol, 198(2):469-78 (2006).
48. Jiang, N., M. Chopp, D. Stein, and H. Feit, Progesterone is neuroprotective after transient middle cerebral artery occlusion in male rats. Brain Res, 735(1):101-7 (1996).
49. Roof, R.L., Duvdevani R Heyburn JW, Stein DG. Progesterone reduces BBB damage following bilateral, medial frontal contusion. in Twenty-first Annual Meeting of the Society for Neuroscience. Miami Beach FL. (1994)
50. Roof, R.L., R. Duvdevani, L. Braswell, and D.G. Stein, Progesterone facilitates cognitive recovery and reduces secondary neuronal loss caused by cortical contusion injury in male rats. Exp Neurol, 129(1):64-9 (1994).
51. Ghoumari, A.M., C. Ibanez, M. EI-Etr, P. Leclerc, B. Eychenne, B.W. O'Malley, E.E. Baulieu, and M. Schumacher, Progesterone and its metabolites increase myelin basic protein expression in organotypic slice cultures of rat cerebellum. J Neurochem, 86(4):848-59 (2003).
52. Ibanez, C., S.A. Shields, M. EI-Etr, E. Leonelli, V. Magnaghi, W.W. Li, F.J. Sim, E.E. Baulieu, R.C. Melcangi, M. Schumacher, and R.J. Franklin, Steroids and the reversal of age-associated changes in myelination and remyelination. Prog Neurobiol, 71(1):49-56 (2003).
53. Gibson, C.L., L.J. Gray, P.M. Bath, and S.P. Murphy, Progesterone for the treatment of experimental brain injury; a systematic review. Brain, 131(Pt 2):318-28 (2008).
54. Sayeed, I. and D.G. Stein, Progesterone as a Neuroprotective Factor in Traumatic and Ischemic Brain Injury, in Neurotherapy, J. Verhaagen, et al., Editors., Elsevier: New York. in press (2009)
55. Stein, D.G. and P.D. Hum, Effects of Sex Steroids on Damaged Neural Systems, in Hormones, Brain and Behavior, D.W. Pfaff, et al., Editors., Elsevier: Oxford. p. 2223-2258 (2009).
56. Wright, D.W., A.L. Kellermann, V.S. Hertzberg, P.L. Clark, M. Frankel, F.C. Goldstein, J.P. Salomone, L.L. Dent, O.A. Harris, D.S. Ander, D.W. Lowery, M.M. Patel, D.D. Denson, A.B. Gordon, M.M. Wald, S. Gupta, S.W. Hoffman, and D.G. Stein, ProTECT: A Randomized Clinical Trial of Progesterone for Acute Traumatic Brain Injury. Ann Emerg Med, 49 (4):391-402 (2007)
57. Xiao, G., J. Wei, W. Yan, W. Wang, and Z. Lu, Improved outcomes from the administration of progesterone for patients with acute severe traumatic brain injury: a randomized controlled trial. Crit Care, 12(2):R61 (2008).
58. Sayeed, I., Q. Guo, S.W. Hoffman, and D.G. Stein, Allopregnanolone, a progesterone metabolite, is more effective than progesterone in reducing cortical infarct volume after transient middle cerebral artery occlusion. Ann Emerg Med, 47(4):381-9 (2006).
59. Sayeed, I., B. Wali, and D.G. Stein, Progesterone inhibits ischemic brain injury in a rat model of permanent middle cerebral artery occlusion. Restor Neurol Neurosci, 25(2):151-9 (2007).
60. Gonzalez-Vidal, M.D., M. Cervera-Gaviria, R. Ruelas, A. Escobar, G. Morali, and M. Cervantes, Progesterone: protective effects on the cat hippocampal neuronal damage due to acute global cerebral ischemia. Arch Med Res, 29(2):117-24 (1998).
61. Cervantes, M., M.D. Gonzalez-Vidal, R. Ruelas, A. Escobar, and G. Morali, Neuroprotective effects of progesterone on damage elicited by acute global cerebral ischemia in neurons of the caudate nucleus.Arch Med Res, 33(1):6-14 (2002).
62. Chen, J., M. Chopp, and Y. Li, Neuroprotective effects of progesterone after transient middle cerebral artery occlusion in rat. J Neurol Sci, 171(1):24-30 (1999).
63. Baulieu, E.E., P. Robel, and M. Schumacher, eds. Neurosteroids A new regulatory function in the Nervous System., Humana Press Inc.: Totawa, New Jersey. 396 (1999).
64. Jung-Testas, I. and E.E. Baulieu, Steroid hormone receptors and steroid action in rat glial cells of the central and peripheral nervous system. J Steroid Biochem Mol Biol, 65(1-6):243-51 (1998).
65. Leonelli, E., R. Bianchi, G. Cavaletti, D. Caruso, D. Crippa, L.M. Garcia-Segura, G. Lauria, V. Magnaghi, I. Roglio, and R.C. Melcangi, Progesterone and its derivatives are neuroprotective agents in experimental diabetic neuropathy: A multimodal analysis. Neuroscience, 144(4):1293-304 (2007).
66. Garay, L., M.C. Deniselle, A. Lima, P. Roig, and A.F. De Nicola, Effects of progesterone in the spinal cord of a mouse model of multiple sclerosis. J Steroid Biochem Mol Biol, 107(3-5):228-37 (2007).
67. Kuebler, J.F., D. Jarrar, K.I. Bland, L. Rue, 3rd, P. Wang, and I.H. Chaudry, Progesterone administration after trauma and hemorrhagic shock improves cardiovascular responses. Crit Care Med, 31(6):1786-93 (2003).
68. Milner, B., P.S. Goldman, G.E. Schneider, and G.S. Lynch, Developmental processes in neural plasticity.Neurosci Res Program Bull, 12(2):212-233 (1974).
69. Schumacher, M., R. Guennoun, D.G. Stein, and A.F. De Nicola, Progesterone: therapeutic opportunities for neuroprotection and myelin repair. Pharmacol Ther, 116(1):77-106 (2007).
70. Traumatic Brain Injury in Prisons and Jails: An Unrecognized Problem at http://www.cdc.gov/traumaticbraininjury/pdf/Prisoner TBI_Prof-a.pdf
71. Get the Stats on Traumatic Brain Injury in the United States at http://www.cdc.gov/traumaticbraininjury/pdf/BlueBook_factsheet-a.pdf
72. Victimization of Persons with Traumatic Brain Injury or Other Disabilities: A Fact Sheet for Professionals at http://www.cdc.gov/trau maticbraininjury/pdfNictimizationTBI Facr/020Sheet4Pros-a.pdf
73. Victimization of Persons with Traumatic Brain Injury or Other Disabilities: A Fact Sheet for Friends and Families at http://www.cdc.gov/trau maticbraini njury/pdfNictimization Person Facr/020Sheet4FrdsFa 1-a.pdf
74. Report to Congress onTraumatic Brain Injury in the United States: Understanding the Public Health Problem among Current and Former Military Personnel (June 2013) at http://www.cdc.gov/trau maticbraininjury/pdf/Report_to_Congress_on Traumatic Brain_1 njury_2013-a.pdf
75. Schatz P, Moser RS.: Current issues in pediatric sports concussion. Clin Neuropsychol; 25:1042-57 (2011).
76. Zhao L, Han W, Steiner C.: Sports related concussions, Statistical brief no. 114. Rockville, MD (2008):
77. Agency for Healthcare Research and Quality, Healthcare Cost and Utilization Project; Available at http://www.hcup-us.ahrq.gov/reports/statbriefs/sb114.jspExternal Web Site Icon. (2011) Accessed October 5, 2011.
78. CDC. Nonfatal traumatic brain injuries from sports and recreation activities--United States, 2001-2005. MMWR; 56:733-7 (2007).
79. McCrory P, Meeuwisse W, Johnston K, et al.: Consensus statement on concussion in sport---the 3rd International Conference on Concussion in Sport, held in Zurich, November 2008. J Clin Neurosci;16:755-63 (2009). Schroeder T, Ault K.: eds. The NEISS sample (design and implementation): 1997 to present. Bethesda, MD: US Consumer Product Safety Commission; Available at http://www.cpsc.govineiss/2001d011-6b6.pdf Adobe PDF fileExternal Web Site Icon (2001). Accessed October 4, 2011.
80. Proctor MR, Cantu RC.: Head and neck injuries in young athletes. Clin Sports Med; 19:693-715 (2000)
81. CDC. Concussion in sports: what can I do to prevent concussions? Atlanta, GA: US Department of Health and Human Services, CDC (2011). Available at http://www.cdc.gov/concussion/sports/prevention.html. Accessed October 3, 2011. 93
82. Buzzini SR, Guskiewicz KM.: Sport-related concussion in the young athlete. Curr Opin Pediatr;18:376-82 (2006).
83. Langlois JA, Rutland-Brown W, Wald MM: The epidemiology and impact of traumatic brain injury. J Head Trauma Rehabil; 21:375-8 (2006).

## Claims

1. A compound of Formula J: 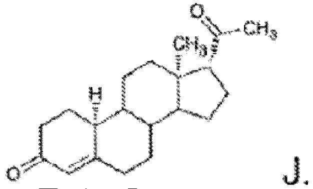 or a pharmaceutically acceptable salt or co-crystal thereof.

2. A pharmaceutical composition comprising a therapeutically useful amount of a compound of Formula J 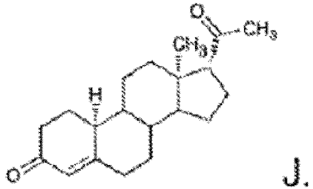 or a pharmaceutically acceptable salt or co-crystal thereof.

3. The pharmaceutical composition of claim 2 wherein said pharmaceutical composition further comprises an additional therapeutic agent selected from the classes consisting of antibodies, proteins and enzymes.

4. The pharmaceutical composition of claim 3 wherein said additional therapeutic agent is a neuroprotective agent, an anti-inflammatory agent, an anti-amyloid agent or an anti-Tau agent.

5. The pharmaceutical composition of claim 2, wherein said pharmaceutical composition is a formulation selected from the list consisting of a tablet, capsule, gelcap, caplet, powder, solution, suspension, eyedrop, cream, lotion, gel and suppository.

6. The pharmaceutical composition of claim 5 wherein said formulation is a powder, a gel or a solution.

7. The pharmaceutical composition of claim 2, wherein the composition is provided as a solution of the compound of Formula J.

8. The pharmaceutical composition of claim 7, wherein the composition is provided as a solution of the compound of Formula J in 2-hydroxypropyl-β-cyclodextrin.

9. The pharmaceutical composition of claim 8, wherein the compound of Formula J has a concentration of about 23mg/ml.

10. The pharmaceutical composition as claimed in any one of claims 2 to 10 for use in the treatment, minimization or prevention of traumatic brain injury, TBI, in a human or animal in need of TBI treatment.

11. The pharmaceutical composition for use as specified in claim 10, wherein the dosage of the pharmaceutical compositions is at least 4mg/Kg of the compound of formula J.

## Patentansprüche

1. Verbindung der Formel J: 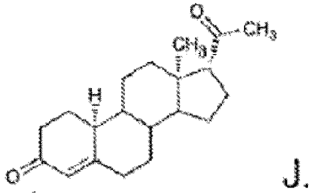 oder ein pharmazeutisch verträgliches Salz oder ein Co-Kristall davon.

2. Pharmazeutische Zusammensetzung, umfassend eine therapeutisch nützliche Menge einer Verbindung der Formel J 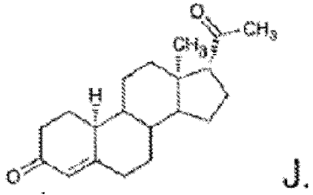 oder ein pharmazeutisch verträgliches Salz oder ein Co-Kristall davon.

3. Pharmazeutische Zusammensetzung nach Anspruch 2, wobei die pharmazeutische Zusammensetzung ferner ein zusätzliches therapeutisches Mittel umfasst, das aus den Klassen ausgewählt ist, die aus Antikörpern, Proteinen und Enzymen bestehen.

4. Pharmazeutische Zusammensetzung nach Anspruch 3, wobei das zusätzliche therapeutische Mittel ein neuroprotektives Mittel, ein entzündungshemmendes Mittel, ein Anti-Amyloid-Mittel oder ein Anti-Tau-Mittel ist.

5. Pharmazeutische Zusammensetzung nach Anspruch 2, wobei die pharmazeutische Zusammensetzung eine Formulierung ist, die aus der Liste ausgewählt ist, die aus einer Tablette, Kapsel, Gelkappe, Kapsel, Pulver, Lösung, Suspension, Augentropfen, Creme, Lotion, Gel und Zäpfchen besteht.

6. Pharmazeutische Zusammensetzung nach Anspruch 5, wobei die Formulierung ein Pulver, ein Gel oder eine Lösung ist.

7. Pharmazeutische Zusammensetzung nach Anspruch 2, wobei die Zusammensetzung als Lösung der Verbindung der Formel J bereitgestellt wird.

8. Pharmazeutische Zusammensetzung nach Anspruch 7, wobei die Zusammensetzung als Lösung der Verbindung der Formel J in 2-Hydroxypropyl-β-Cyclodextrin bereitgestellt wird.

9. Pharmazeutische Zusammensetzung nach Anspruch 8, wobei die Verbindung der Formel J eine Konzentration von etwa 23 mg / ml aufweist.

10. Pharmazeutische Zusammensetzung nach einem der Ansprüche 2 bis 10 zur Verwendung bei der Behandlung, Minimierung oder Prävention von traumatischer Hirnverletzung, TBI, bei einem Menschen oder Tier, der oder das eine TBI-Behandlung benötigt.

11. Pharmazeutische Zusammensetzung zur Verwendung nach Anspruch 10, wobei die Dosierung der pharmazeutischen Zusammensetzungen mindestens 4 mg / kg der Verbindung der Formel J beträgt.

## Revendications

1. Composé de formule J : 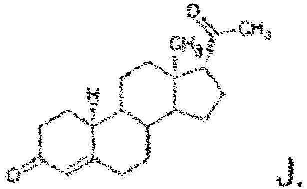 ou un sel ou co-cristal pharmaceutiquement acceptable de celui-ci.

2. Composition pharmaceutique comprenant une quantité thérapeutiquement utile d'un composé de formule J 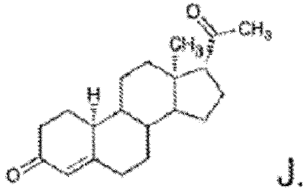 ou un sel ou co-cristal pharmaceutiquement acceptable de celui-ci.

3. Composition pharmaceutique selon la revendication 2, dans laquelle ladite composition pharmaceutique comprend en outre un agent thérapeutique supplémentaire choisi parmi les classes constituées des anticorps, des protéines et des enzymes.

4. Composition pharmaceutique selon la revendication 3, dans laquelle ledit agent thérapeutique supplémentaire est un agent neuroprotecteur, un agent anti-inflammatoire, un agent anti-amyloïde ou un agent anti-Tau.

5. Composition pharmaceutique selon la revendication 2, dans laquelle ladite composition pharmaceutique est une formulation choisie dans la liste constituée d'un comprimé, d'une capsule, d'une gellule, d'un caplet, d'une poudre, d'une solution, d'une suspension, d'un collyre, d'une crème, d'une lotion, d'un gel et d'un suppositoire.

6. Composition pharmaceutique selon la revendication 5, dans laquelle ladite formulation est une poudre, un gel ou une solution.

7. Composition pharmaceutique selon la revendication 2, dans laquelle la composition est fournie sous forme d'une solution du composé de formule J.

8. Composition pharmaceutique selon la revendication 7, dans laquelle la composition est fournie sous forme d'une solution du composé de formule J dans : 2-hydroxypropyl-β-cyclodextrine.

9. Composition pharmaceutique selon la revendication 8, dans laquelle le composé de formule J a une concentration d'environ 23 mg/ml.

10. Composition pharmaceutique selon l'une quelconque des revendications 2 à 10 destinée à être utilisée dans le traitement, la minimisation ou la prévention des lésions cérébrales traumatiques, TBI, chez un être humain ou animal nécessitant un traitement TBI.

11. Composition pharmaceutique destinée à être utilisée selon la revendication 10, dans laquelle le dosage des compositions pharmaceutiques est d'au moins 4 mg/kg du composé de formule J.
